# EUROPEAN PATENT SPECIFICATION

(11) **EP 0 882 734 B1**
(45) Date of publication and mention of the grant of the patent: **26.08.2009**
(21) Application number: 98109318.0
(22) Date of filing: 22.05.1998
(51) Int. Cl.: C07H 19/067, A61K 31/70

(54) **5'-Deoxy-cytidine derivatives**
5-Deoxy-Cytidin-Derivate
Dérivés de 5'-déoxy-cytidine

(30) Priority: 02.06.1997 EP 97108791
(43) Date of publication of application: 09.12.1998
(73) Proprietor: F. Hoffmann-La Roche AG, 4070 Basel (CH)
(72) Inventor: Hattori, Kazuo, Kanagawa-ken (JP); Ishikawa, Tohru, Kanagawa-ken (JP); Ishitsuka, Hideo, Yokohama-shi, Kanagawa-ken (JP); Kohchi, Yasunori, Fujisawa-shi, Kanagawa-ken (JP); Oikawa, Nobuhiro, Yokohama-shi, Kanagawa-ken (JP); Shimma, Nobuo, Chigasaki-shi, Kanagawa-ken (JP); Suda, Hitomi, Fujisawa-shi, Kanagawa-ken 252 (JP)

(56) References cited:
- EP-A- 0 316 704
- EP-A- 0 602 454
- EP-A- 0 602 478
- EP-A- 0 712 629
- WO-A-85/01871
- WO-A-94/26761
- WO-A-95/07917
- WO-A-96/37214
- WO-A-99/40099
- US-A- 4 395 406
- M.KRECMEROVA AT AL.: "Preparation of 5-Benzyluracil and 5-Benzylcytosine Nucleosides as Potential Inhibitors of Uridine Phosphorylase." COLLECTION OF CZECHOSLOVAK CHEMICAL COMMUNICATIONS., vol. 61, no. 4, April 1996 (1996-04), pages 627-644, XP002090902 PRAGUE CS

## Description

The present invention is concerned with novel 5'-deoxy-cytidine derivatives, pharmaceutical compositions, a kit thereof for assisting a delivery of 5-fluorouracil selectively to tumor tissues and process for manufacturing the novel 5'-deoxy-cytidine derivatives.

More particularly, the present invention relates to novel 5'-deoxy-cytidine derivatives represented by the general formula (I), 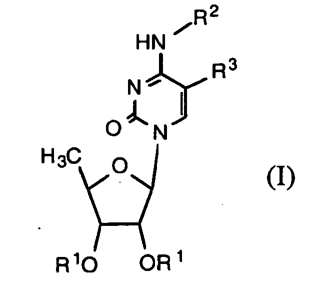 wherein R¹ is a hydrogen atom or a group selected from acetyl, propionyl, benzoyl, toluoyl, glycyl, alanyl, β-alanyl, valyl, lysyl; R² is a hydrogen atom, or -CO-OR⁴ group [wherein R⁴ is a saturated or unsaturated, straight or branched hydrocarbon group consisting of one to fifteen carbon atoms, or a group of the formula -(CH₂)ₙ-Y (in which Y is cyclohexyl or phenyl; n is an integer from 0 to 4)]; R³ is a hydrogen atom, bromo, iodo, trifluoromethyl, ethyl, propyl, cyano, vinyl, 1-chlorovinyl, ethynyl, prop-1-ynyl, but-1-ynyl, pent-1-ynyl, hex-1-ynyl, or bromoethynyl; with the proviso that R² and R³ do not mean a hydrogen atom at the same time.

Although 5-fluorouracil (5-FU) or its derivatives are clinically useful antitumor agents for the treatment of various solid tumors, in general they are still not satisfactory in terms of efficacy and safety. These drawbacks are mainly due to rapid inactivation of 5-FU by dihydropyrimidine dehydrogenase (DPD) and/or the unsatisfactory delivery of 5-FU to tumor tissues with respect to tumor selectivity. The attempts to enhance the antitumor activity of 5-FU or its derivatives by inhibition of DPD have already been reported : the co-administration of 5-FU or its derivative with a DPD inhibitor such as uracil [USP 4,328,229], 5-ethynyluracil [WO92/04901], 5-chloro-2,4-dihydroxypyridine [USP 5,525,603] etc. Such co-administration resulted in enhancement of the antitumor activity of 5-FU or its derivatives, but the safety profile was not so improved due to insufficient selectivity in delivering the DPD inhibitor to tumor tissues (as a consequence, 5-FU level is increased both in tumor and plasma).

In contrast, in accordance with the present invention it has been found that the co-administration of a novel 5'-deoxy-cytidine derivative represented by the general formula (I) with 5-FU or its derivative results in the significantly improved delivery of 5-FU selectively to tumor tissues as compared with the combination of 5-FU or its derivative with a known DPD inhibitor such as 5-ethynyluracil, and shows significantly improved antitumor activity in human cancer xenograft models.

The respective groups of the general formula (I) are explained in more detail as follows;
Explanation of R¹:
   R¹ is a hydrogen atom or a group selected from acetyl, propionyl, benzoyl, toluoyl, glycyl, alanyl, β-alanyl, valyl, lysyl.
Explanation of R²:
   R² is a hydrogen atom, or -CO-OR⁴ group [wherein R⁴ is a saturated or unsaturated, straight or branched hydrocarbon group consisting of one to fifteen carbon atoms, or group of formura -(CH₂)ₙ-Y (in which Y is cyclohexyl or phenyl; n is an integer of from 0 to 4)].

   In the above group R⁴, the term "a saturated or unsaturated, straight or branched hydrocarbon group consisting of one to fifteen carbon atoms" preferably means methyl, ethyl, n-propyl, 1-isopropyl-2-methylpropyl, 1,1,2-trimethylpropyl, n-butyl, isobutyl, 2-ethylbutyl, 3,3-dimethylbutyl, n-pentyl, isopentyl, neopentyl, 2-propylpentyl, n-hexyl, 2-ethylhexyl, n-heptyl, n-octyl, allyl, 2-buten-1-yl, 3-buten-1-yl, 3-penten-1-yl, 4-penten-1-yl, 3-hexen-1-yl, 4-hexen-1-yl, 5-hexen-1-yl, n-tridecyl and the like.
   The term "a group of the formula -(CH₂)ₙ-Y (in which Y is cyclohexyl or phenyl; n is an integer from 0 to 4)" preferably means cyclohexyl, cyclohexylmethyl, 2-cyclohexylethyl, 3-cyclohexylpropyl, 4-cyclohexyl-butyl, phenyl, benzyl, phenethyl, 3-phenylpropyl, 4-phenylbutyl and the like. In the most preferred embodiment of the compounds in accordance with the present invention, R⁴ means n-propyl, n-butyl, n-pentyl, isopentyl, neopentyl, n-hexyl, 3,3-dimethylbutyl, 2-ethylbutyl, phenylethyl, and cyclohexylmethyl.
Explanation of R³:
   R³ is a hydrogen atom, bromo, iodo, trifluoromethyl, ethyl, propyl, cyano, vinyl, 1-chlorovinyl, ethynyl, prop-1-ynyl, but-1-ynyl, pent-1-ynyl, hex-1-ynyl, or bromoethynyl; with the proviso that R² and R³ do not mean a hydrogen atom at the same time.

Preferred 5'-deoxy-cytidine derivatives of the present invention are:
5'-deoxy-5-ethynylcytidine,
5'-deoxy-5-prop-1-ynylcytidine,
5-but-1-ynyl-5'-deoxycytidine,
5'-deoxy-5-pent-1-ynylcytidine,
5'-deoxy-5-hex-1-ynylcytidine,
5'-deoxy-5-iodocytidine,
5-bromo-5'-deoxycytidine,
5-(1-chlorovinyl)-5'-deoxycytidine,
5'-deoxy-5-vinylcytidine,
5'-deoxy-5-trifluoromethylcytidine 5-cyano-5'-deoxycytidine,
5'-deoxy-N⁴-(n-pentyloxycarbonyl)cytidine,
5'-deoxy-N⁴-(n-pentyloxycarbonyl)-5-prop-1-ynylcytidine,
5-but-1-ynyl-5'-deoxy-N⁴-(n-pentyloxycarbonyl)cytidine,
5'-deoxy-5-pent-1-ynyl-N⁴-(n-pentyloxycarbonyl)cytidine,
5'-deoxy-5-hex-1-ynyl-N⁴-(n-pentyloxycarbonyl)cytidine,
5'-deoxy-5-iodo-N⁴-(n-pentyloxycarbonyl)cytidine,
5-bromo-5'-deoxy-N⁴-(n-pentyloxycarbonyl)cytidine,
5-(1-chlorovinyl)-5'-deoxy-N⁴-(n-pentyloxycarbonyl)cytidine,
N⁴-(ethoxycarbonyl)-5'-deoxy-5-vinylcytidine,
5'-deoxy-N⁴-(n-propoxycarbonyl)-5-vinylcytidine,
N⁴-(n-butoxycarbonyl)-5'-deoxy-5-vinylcytidine,
5'-deoxy-N⁴-(n-pentyloxycarbonyl)-5-vinylcytidine,
N⁴-(benzyloxycarbonyl)-5'-deoxy-5-vinylcytidine,
5'-deoxy-N⁴-(n-pentyloxycarbonyl)-5-trifluoromethylcytidine, 5-cyano-5'-deoxy-N⁴-(n-pentyloxycarbonyl)cytidine,
5'-deoxy-5-ethynyl-N⁴-(methoxycarbonyl)cytidine
5'-deoxy-N⁴-(ethoxycarbonyl)-5-ethynylcytidine
5'-deoxy-5-ethynyl-N⁴-(n-propoxycarbonyl)cytidine,
5'-deoxy-5-ethynyl-N⁴-(isopropoxycarbonyl)cytidine,
N⁴-(n-butoxycarbonyl)-5'-deoxy-5-ethynylcytidine,
5'-deoxy-5-ethynyl-N⁴-(isobutoxycarbonyl)cytidine,
5'-deoxy-5-ethynyl-N⁴-(n-pentyloxycarbonyl)cytidine,
5'-deoxy-5-ethynyl-N⁴-[(2-propylpentyloxy)carbonyl]cytidine,
5'-deoxy-5-ethynyl-N⁴-(isopentyloxycarbonyl)cytidine,
5'-deoxy-5-ethynyl-N⁴-[(2-methylpentyloxy)carbonyl]cytidine,
5'-deoxy-5-ethynyl-N⁴-[(3-methylpentyloxy)carbonyl]cytidine,
5'-deoxy-5-ethynyl-N⁴-(n-hexyloxycarbonyl)cytidine,
5'-deoxy-N⁴-[(2-ethylbutyl)oxycarbonyl]-5-ethynylcytidine,
5'-deoxy-N⁴-[(2-ethylhexyl)oxycarbonyl]-5-ethynylcytidine,
5'-deoxy-5-ethynyl-N⁴-[(2-phenylethoxy)carbonyl]cytidine,
N⁴-(cyclohexyloxycarbonyl)-5'-deoxy-5-ethynylcytidine,
N⁴-[(cyclohexylmethoxy)carbonyl]-5'-deoxy-5-ethynylcytidine,
5'-deoxy-5-ethynyl-N⁴-(neopentyloxycarbonyl)cytidine,
5'-deoxy-N⁴-[(3,3-dimethylbutoxy)carbonyl]-5-ethynylcytidine,
2',3'-di-O-acetyl-5'-deoxy-5-ethynyl-N⁴-(n-propoxycarbonyl)cytidine
2',3'-di-O-acetyl-5'-deoxy-5-ethynyl-N⁴-(n-pentyloxycarbonyl)cytidine
2',3'-di-O-acetyl-N⁴-(ethoxycarbonyl)-5'-deoxy-5-vinylcytidine
2',3'-di-O-acetyl-5'-deoxy-N⁴-(n-propoxycarbonyl)-5-vinylcytidine,
2',3'-di-O-acetyl-N⁴-(n-butoxycarbonyl)-5'-deoxy-5-vinylcytidine,
2',3'-di-O-acetyl-5'-deoxy-N⁴-(n-pentyloxycarbonyl)-5-vinylcytidine,
2',3'-di-O-acetyl-N⁴-(benzyloxycarbonyl)-5'-deoxy-5-vinylcytidine.
5'-deoxy-5-ethynyl-N⁴-(n-decyloxycarbonyl)cytidine
5'-deoxy-5-ethynyl-N⁴-[(2,6-dimethylcyclohexyloxy)-carbonyl]cytidine
5'-deoxy-5-ethynyl-N⁴-(benzyloxycarbonyl)cytidine
5'-deoxy-5-ethynyl-N⁴-[(1-isopropyl-2-methyl-propoxy)carbonyl]cytidine
5'-deoxy-5-ethynyl-N⁴-[(3-methoxybenzyloxy)-carbonyl]cytidine.

The novel 5'-deoxy-cytidine derivatives represented by the formula (I) can be produced according to the following methods. In the following process A-F, P¹ represents a hydroxy protecting group such as acetyl, benzoyl, trimethylsilyl, tert-butyldimethylsilyl and the like.

### Process A:

Compounds represented by the formula (I) wherein R¹, R² and R³ are the same as defined above can be prepared by reacting a compound represented by the formula (II) 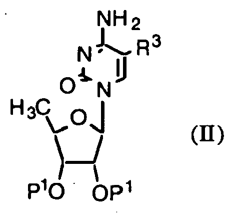 [wherein P¹ is a hydroxy-protecting group, and R³ is the same as defined above],
with a compound represented by the general formula (III),

R⁴OCOX (III)

[wherein R⁴ is the same as defined above; X is chloro or bromo], in the presence of acid acceptor, followed, if necessary, by removal of protecting group(s),

### Process B:

Compounds represented by the formula (I), wherein R¹ and R² are the same as defined above and R³ is vinyl, 1-chlorovinyl, ethynyl, prop-1-ynyl, but-1-ynyl, pent-1-ynyl, hex-1-ynyl, or bromoethynyl, can also be prepared by reacting a compound represented by the formula (IV) 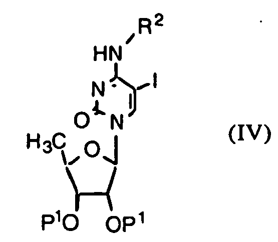 [wherein P¹ and R² are the same as defined above],
with an acetylene or vinyl derivative in the presence of a palladium catalyst, followed, if necessary, by removal of protecting group(s).

### Process C:

Compounds represented by the formula.(I), wherein R¹ and R² are the same as defined above and R³ is a cyano group, can be prepared by reacting a compound represented by the formula (IV) 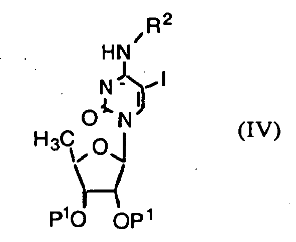 [wherein P¹ and R² is the same as defined above],
with alkali metal cyanide, followed, if necessary, by removal of protecting group(s).

### Process D:

Compounds represented by the formula (I), wherein R¹ and R³ are the same as defined above and R² is a hydrogen atom, can also be prepared by reacting a compound represented by the formula (V) 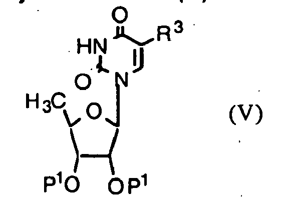 [wherein P¹ and R³ are the same as defined above],
with phosphoryl chloride in the presence of an acid acceptor, followed by treatment with ammonia,
followed, if necessary, by removal of protecting group(s).

### Process E:

Compounds represented by the formula (I), wherein R¹, R² and R³ are the same as defined above, can also be prepared by coupling a compound represented by the formula (VI) 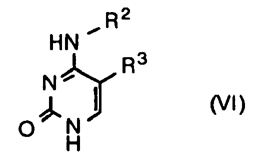 [wherein R² and R³ are the same as defined above],
with a compound represented by the formula (VII) 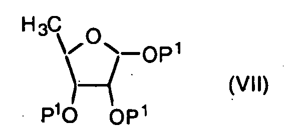 [wherein P¹ is the same as defined above]
in the presence of Lewis acid catalyst, followed, if necessary, by removal of protecting group(s).

### Process F:

Compounds represented by the formula (I) wherein R³ is vinyl, 1-chlorovinyl, R¹and R² are the same as defined above can be prepared by catalytic hydrogenation of a compound represented by the formula (VIII) 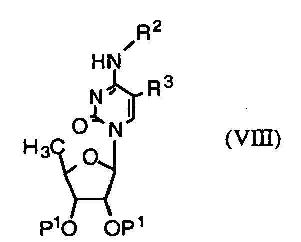 [wherein P¹ is a hydroxy-protecting radical, R³ is an ethynyl or 1-chloroethynyl radical, and R² is the same as defined above],
with Lindlar catalyst, followed, if necessary, by removal of protecting radical(s).

In the following, process for producing novel 5'-deoxy-cytidine derivatives represented by the formula (I) according to the present invention will be explained in more detail.

### Process A:

Specific examples of the compounds represented by the general formula (II) include,
2',3'-di-O-acetyl-5'-deoxy-5-ethynylcytidine,
2',3'-bis-O-(tert-butyldimethylsilyl)-5'-deoxy-5-ethynylcytidine,
2',3'-di-O-acetyl-5'-deoxy-5-prop-1-ynylcytidine,
2',3'-bis-O-(tert-butyldimethylsilyl)-5'-deoxy-5-prop-1-ynylcytidine,
2',3'-di-O-acetyl-5-but-1-ynyl-5'-deoxycytidine,
2',3'-bis-O-(tert-butyldimethylsilyl)-5-but-1-ynyl-5'-deoxycytidine,
2',3'-di-O-acetyl-5'-deoxy-5-pent-1-ynylcytidine,
2',3'-bis-O-(tert-butyldimethylsilyl)-5'-deoxy-5-pent-1-ynylcytidine,
2',3'-di-O-acetyl-5'-deoxy-5-hex-1-ynylcytidine,
2',3'-bis-O-(tert-butyldimethylsilyl)-5'-deoxy-5-hex-1-ynylcytidine,
2',3'-di-O-acetyl-5'-deoxy-5-iodocytidine,
2',3'-bis-O-(tert-butyldimethylsilyl)-5'-deoxy-5-iodocytidine,
2',3'-di-O-acetyl-5-bromo-5'-deoxycytidine,
2',3'-bis-O-(tert-butyldimethylsilyl)-5-bromo-5'-deoxycytidine,
2',3'-di-O-acetyl-5-(1-chlorovinyl)-5'-deoxycytidine,
2',3'-bis-O-(tert-butyldimethylsilyl)-5-(1-chlorovinyl)-5'-deoxycytidine,
2',3'-bis-O-(tert-butyldimethylsilyl)-5'-deoxy-5-vinylcytidine,
2',3'-di-O-acetyl-5'-deoxy-5-trifluoromethylcytidine,
2',3'-bis-O-(tert-butyldimethylsilyl)-5'-deoxy-5-trifluoromethylcytidine,
2',3'-di-O-acetyl-5-(3-benzyloxybenzyl)-5'-deoxycytidine,
2',3'-di-O-acetyl-5-cyano-5'-deoxycytidine,
2',3'-bis-O-(tert-butyldimethylsilyl)-5-cyano-5'-deoxycytidine
and the like.

The reaction of the compound of the above general formula (II) with the compound of the above general formula (III) can be carried out in a solvent such as pyridine, dioxane, tetrahydrofuran, acetonitrile, chloroform, dichloromethane and the like in the presence of an acid acceptor such as triethylamine, pyridine, picoline, 4-(N,N-dimethylamino)pyridine, lutidine and the like. The reaction can be carried out at a temperature between 0 and 30°C. The protecting group(s) may, if necessary, be removed after the reaction by the procedures known to those skilled in the art, e.g. by basic or acidic hydrolysis, or treatment with fluoride anion.

### Process B:

Specific examples of the compound represented by the general formula (IV) include,
2',3'-bis-O-(tert-butyldimethylsilyl)-5'-deoxy-5-iodo-N⁴-(methoxycarbonyl)cytidine,
2',3'-bis-O-(tert-butyldimethylsilyl)-5'-deoxy-N⁴-(ethoxycarbonyl)-5-iodocytidine,
2',3'-bis-O-(tert-butyldimethylsilyl)-5'-deoxy-5-iodo-N⁴-(n-propoxycarbonyl)-cytidine,
N⁴-(n-butoxycarbonyl)-2',3'-bis-O-(tert-butyldimethylsilyl)-5'-deoxy-5-iodocytidine,
2',3'-bis-O-(tert-butyldimethylsilyl)-5'-deoxy-5-iodo-N⁴-(n-pentyloxy-carbonyl)-cytidine,
2',3'-bis-O-(tert-butyldimethylsilyl)-5'-deoxy-5-iodo-N⁴-(isopentyloxy-carbonyl)-cytidine,
2',3'-bis-O-(tert-butyldimethylsilyl)-5'-deoxy-5-iodo-N⁴-(n-hexyloxy-carbonyl)-cytidine,
2',3'-bis-O-(tert-butyldimethylsilyl)-5'-deoxy-N⁴-[(2-ethylbutyl)oxycarbonyl]-5-iodocytidine,
2',3'-bis-O-(tert-butyldimethylsilyl)-5'-deoxy-5-iodo-N⁴-[(2-phenylethoxy)-carbonyl]cytidine,
2',3'-bis-O-(tert-butyldimethylsilyl)-N⁴-[(cyclohexylmethoxy)carbonyl]-5'-deoxy-5-iodocytidine,
2',3'-bis-O-(tert-butyldimethylsilyl)-5'-deoxy-5-iodo-N4-(neopentyloxycarbonyl)cytidine,
2',3'-bis-O-(tert-butyldimethylsilyl)-5'-deoxy-N4-[(3,3-dimethylbutoxy)-carbonyl]-5-iodocytidine,
2',3'-di-O-acetyl-5'-deoxy-5-iodo-N⁴-(ethoxycarbonyl)-cytidine,
2',3'-di-O-acetyl-5'-deoxy-5-iodo-N⁴-(n-propoxycarbonyl)cytidine
2',3'-di-O-acetyl-N⁴-(n-butoxycarbonyl)-5'-deoxy-5-iodo-cytidine,
2',3'-di-O-acetyl-5'-deoxy-5-iodo-N⁴-(n-pentyloxycarbonyl)cytidine
and the like.

Specific examples of the acetylene or vinyl derivatives used for this coupling reaction are trimethysilyl acetylene, tert-butyldimethysilyl acetylene, 1-butyne, 1-pentyne, 1-heptyne, 1-hexyne, 3-methyl-1-butyne, 3,3-dimethyl-1-butyne, cyclohexylacetylene, phenylacetylene, 3-phenyl-1-propyne, tri-n-butyl(vinyl)stannane and the like.

The coupling reaction of a compound represented by the formula (IV) with an acetylene derivative can be performed in the presence of a palladium catalyst such as bis(triphenylphosphine)palladium (II) chloride-copper (I) iodide, bis(triphenylphosphine)palladium(II) acetate-copper(I) iodide and the like. The coupling reaction of a compound represented by the formula (IV) with a vinyl derivative can be performed in the presence of palladium catalyst such as tris(dibenzylideneacetone)dipalladium, tetrakis(triphenylphosphine)palladium, bis(acetonitrile)palladium (II) chloride in the presence of tri-2-furylphosphine, triphenylphosphine and the like.
These reaction can be carried out in a solvent such as chloroform, dichloromethane, tetrahydrofurane, N-methylpyrrolidone, N,N-dimethyformamide and the like. The reaction can be carried out at a temperature between 0 and 80°C, preferably between 10 and 60 °C.

### Process C:

The reaction of the compound of the above general formula (IV) with alkali metal cyanide such as sodium cyanide, potassium cyanide etc. can be carried out in a solvent such as N,N-dimethylformamide, dimethylsulfoxide, acetonitrile and the like. The reaction can be carried out at a temperature between 0 and 100°C, preferably between 10 and 30°C.

### Process D:

Specific examples of the compounds represented by the general formula (V) include,
2',3'-bis-O-(tert-butyldimethylsilyl)-5'-deoxy-5-ethynyluridine,
2',3'-bis-O-(tert-butyldimethylsilyl)-5'-deoxy-5-prop-1-ynyluridine,
2',3'-bis-O-(tert-butyldimethylsilyl)-5-but-1-ynyl-5'-deoxyuridine,
2',3'-bis-O-(tert-butyldimethylsilyi)-5'-deoxy-5-pent-1-ynyluridine,
2',3'-bis-O-(tert-butyldimethylsilyl)-5'-deoxy-5-hex-1-ynyluridine,
2',3'-bis-O-(tert-butyldimethylsilyl)-5'-deoxy-5-iodouridine,
5-bromo-2',3'-bis-O-(tert-butyldimethylsilyl)-5'-deoxyuridine,
2',3'-bis-O-(tert-butyldimethylsilyl)-5-(1-chlorovinyl)-5'-deoxyuridine,
2',3'-bis-O-(tert-butyldimethylsilyl)-5'-deoxy-5-vinyluridine,
2',3'-bis-O-(tert-butyldimethylsilyl)-5'-deoxy-5-trifluoromethyluridine, 2',3'-bis-O-(tert-butyldimethylsilyl)-5-cyano-5'-deoxyuridine
and the like.

The starting materials listed above can be prepared from the known 5-substituted uracil derivatives by the method similar to the process E wherein 5-substituted uracil derivative is used instead of 5-substituted cytosine derivative.

The reaction of the compound of the above general formula (V) with phosphoryl chloride can be carried out in a solvent such as pyridine, dioxane, tetrahydrofuran, acetonitrile, chloroform, dichloromethane and the like in the presence of acid acceptor such as triethylamine, pyridine, picoline, 4-(N,N-dimethylamino)pyridine, lutidine, imidazole, N-methylimidazole, triazole and the like at a temparature between 0 and 30°C, followed by treatment with aqueous ammonia or ammonia gas in a solvent such as methanol, ethanol, acetonitrile, N,N-dimethylformamide and the like at a temperature between 0 and 30 °C.

### Process E:

Specific examples of the compounds represented by the general formula (VI) include 5-ethynylcytosine, 5-prop-1-ynylcytosine, 5-prop-1-ynylcytosine, 5-but-1-ynyl-5'-deoxycytosine, 5-pent-1-ynylcytosine, 5-hex-1-ynylcytosine, 5-iodocytosine, 5-bromocytosine, 5-(1-chlorovinyl)-cytosine, 5-vinylcytosine, 5-trifluoromethylcytosine, 5-(3-benzyloxy-benzyl)cytosine, 5-cyanocytosine, 5-ethynyl-N⁴-(n-pentyloxycarbonyl)cytosine and the like.

Specific examples of the compound represented by the general formula (VII) include known 5-deoxy-1,2,3-O-triacetyl-D-ribofuranoside, 5-deoxy-1,2,3-O-tribenzoyl-D-ribofuranoside and the like.

A compound of the formula (VI) may be first converted to the trimethylsilyl derivative with silylation reagent such as hexamethyldisilazane, followed by the coupling reaction with a compound represented by the formula (VII) in the presence of Lewis acid catalyst such as tin-(IV)-chloride, titanium-(IV)-chloride and the like. This coupling reaction proceeds in a solvent such as acetonitrile, dichloromethane, chloroform, 1,2-dichloroethane, nitromethane, toluene and the like, at a temperature between 0 and 30 °C, preferably between 0 and 10 °C.

### Process F:

Specific examples of the compounds represented by then general formula (VIII) include
5'-deoxy-5-ethynylcytidine,
5'-deoxy-N⁴-(ethoxycarbonyl)-5-ethynylcytidine
5'-deoxy-5-ethynyl-N⁴-(n-propoxycarbonyl)cytidine,
N⁴-(n-butoxycarbonyl)-5'-deoxy-5-ethynylcytidine,
5'-deoxy-5-ethynyl-N⁴-(n-pentyloxycarbonyl)cytidine,
N⁴-(benzyloxycarbonyl)-5'-deoxy-5-ethynylcytidine,
2',3'-di-O-acetyl-5'-deoxy-5-ethynylcytidine
2',3'-di-O-acetyl-5'-deoxy-5-ethynyl-N⁴-(ethoxycarbonyl)cytidine,
2',3'-di-O-acetyl-5'-deoxy-5-ethynyl-N⁴-(n-propoxycarbonyl)cytidine,
2',3'-di-O-acetyl-5'-deoxy-5-ethynyl-N⁴-(n-pentyloxycarbonyl)cytidine and the like.
The catalytic hydrogenation of the ethynyl group of the compound of formula (VIII) can be performed using Lindlar catalyst according to the method known to those skilled in the art [ cf. Synthetic Method, 1952, vol. 7, P38 (Interscience Publishers, Inc., New York)].

The novel 5'-deoxy-cytidine derivatives of the present invention can be used as antitumor agent together with known physiologically acceptable pharmaceutical carriers.

The present invention also provides a pharmaceutical composition comprising a 5'-deoxy-cytidine derivative represented by the general formula (I) and 5-fluorouracil (5-FU) or a derivative thereof. With this composition, the 5'-deoxy-cytidine derivative potentiates the antitumor effect of 5-fluorouracil or its derivative by delivering a significantly higher amount of 5-FU selectively to tumor tissues without significant increase of 5-FU concentration in plasma.

Especially preferred is a pharmaceutical composition comprising 2',3'-di-O-acetyl-5'-deoxy-5-vinylcytidine and 5-fluorouracil or a derivative thereof.

For the combination of a 5'-deoxy-cytidine derivative represented by the general formula (I) with 5-FU or a derivative thereof for the treatment of cancer with an improved efficacy and safety profile, the 5-FU derivative is preferably selected from the group consisting of:
5-fluoro-1-(2-tetrahydrofuryl)uracil,
1-(n-hexyloxycarbonyl)-5-fluorouracil,
5'-deoxy-5-fluorouridine,
5'-deoxy-5-fluoro-N⁴-(n-propoxycarbonyl)cytidine,
N⁴-(n-butoxycarbonyl)-5'-deoxy-5-fluorocytidine,
5'-deoxy-5-fluoro-N⁴-(n-pentyloxycarbonyl)cytidine,
5'-deoxy-5-fluoro-N⁴-(isopentyloxycarbonyl)cytidine,
5'-deoxy-5-fluoro-N⁴-(n-hexyloxycarbonyl)cytidine,
5'-deoxy-N⁴-[(2-ethylbutyl)oxycarbonyl]-5-fluorocytidine,
5'-deoxy-5-fluoro-N⁴-[(2-phenylethoxy)carbonyl]cytidine,
N⁴-[(cyclohexylmethoxy)carbonyl]-5'-deoxy-5-fluorocytidine,
5'-deoxy-5-fluoro-N⁴-(neopentyloxycarbonyl)-cytidine,
5'-deoxy-N⁴-[(3,3-dimethylbutoxy)carbonyl]-5-fluorocytidine,
5'-deoxy-5-fluoro-N⁴-(3,5-dimethylbenzoyl)cytidine,
5'-deoxy-5-fluoro-N⁴-(3,5-dichlorobenzoyl)cytidine,
2',3'-di-O-acetyl-5'-deoxy-5-fluoro-N⁴-(n-pentyloxycarbonyl)cytidine and the like.

A compound of the formula (I) can be administered either alone or simultaneously with 5-FU or a derivative thereof.

Accordingly, the pharmaceutical composition of the present invention can be obtained by formulating a compound of the formula (I) and 5-FU or a derivative thereof into a single preparation or may be provided in the form of two separate individual preparations.

A pharmaceutical composition of the formula (I) can be administered before or simultaneously with the administration of 5-FU or a derivative thereof; preferably, within 3 hour before or simultaneously with the administration of 5-FU or a derivative thereof.

In the pharmaceutical composition of the present invention comprising 5-FU or a derivative thereof and a 5'-deoxy-cytidine derivative represented by the general formula (I), the suitable molar ratio of two components is about 0.001-10 moles, preferably 0.002-0.5 mole of a compound of the formula (I) per mole of 5-FU or its derivative.

The present invention also provides a kit comprising a pharmaceutical composition (component A) containing a compound of the formula (I) and a pharmaceutical composition (component B) containing 5-FU or a derivative thereof.

Thus, the present invention is also concerned with pharmaceutical compositions of a compound of formula (I) and, optionally, 5-FU or a derivative thereof and a kit thereof for the treatment of colorectal cancer, breast cancer, stomach cancer, lung cancer, cervical cancer, bladder cancer and other malignant diseases and the like.

The pharmaceutical compositions and the components A and B of the kit of the present invention can be administered in any form, for example, tablets, pills, suppositories, capsules, granules, powders, or emulsions etc. Pharmaceutically acceptable carriers and excipients useful in formulating the pharmaceutical composition of the present invention are those commonly used. Pharmaceutically acceptable materials can be an organic or inorganic inert carrier material suitable for enteral, percutaneous or parenteral administration such as water, gelatine, gum arabic, lactose, starch, magnesium stearate, talc, vegetable oils, polyalkylene glycols and petroleum jelly. The pharmaceutical composition provided by the present invention can be administered orally, e.g., in form of tablets, capsules, pills, powders, granules, solutions, syrups, suspensions or elixirs. The administration can also be carried out parenterally, e.g. in form of sterile solutions, suspensions or emulsions; or locally, e.g., in form of solutions, suspensions, salves, powders or aerosols. The pharmaceutical composition can be sterilized and/or can contain further adjuvants such as preserving, stabilizing setting, emulsifying agents, flavor-improving agents , salts for variation of the osmotic pressure or substances acting as buffers.

The pharmaceutical composition can be prepared in a conventional manner.

Dosage ranges for the pharmaceutical composition of the present invention depend on the route of administration, the age, weight and condition of the patient and the particular disease to be treated. In the case of oral, rectal or parenteral administration for adults, an approximate daily dosage is in the range of from about 1 mg to about 2,000 mg of a compound of formula (I) and from about 10 mg to about 4,000 mg of 5-FU or its derivative, depending on the kind of 5-FU derivative used. Oral administration is a preferred route of administration of the pharmaceutical composition according to the present invention.

The tumor selective delivery of 5-FU through the tumor DPD selective inhibition by a compound of formula (I) is evident from the test described hereafter.

### 1. Tumor DPD Selective Inhibition by Compound A of Example 6

The activity of compound A of Example 6 to inhibit DPD activity was compared with that of the known DPD inhibitor 5-ethynyluracil (5-EU) in BALB/c nude mice bearing the human prostate cancer xenograft PC-3. The liver and tumor tissues were resected out from each group of three mice at 2 and 8 hours after the administration of the Compound A (0.5 mmol/kg) and 5-EU (0.05 µmol/kg). DPD activity in these tissues was then measured as described elsewhere (Naguib et al., Cancer Research 45, 5405-5412, 1985). 5-EU inhibited the DPD activity both in the liver and tumor tissue, whereas compound A strongly inhibited the activity only in tumor tissue (Table 1). These results suggest that compound A of Example 6 inhibits DPD activity selectively in tumor tissue.

**Table 1. Inhibition of DPD activity by Compound A of Example 6**

| Tissues | DPD activities (pmol/mg protein/min) | | | | | |
|---|---|---|---|---|---|---|
| | Control | | 5-EU | | Compound A | |
| | 2 hr | 8 hr | 2 hr | 8 hr | 2 hr | 8 hr |
| Liver | 288 | 162 | 46 | 83 | 177 | 326 |
| Tumor | 31 | 29 | 17 | 13 | 9 | 9 |

### 2. Selective increase of 5-FU levels in tumors by compound A of Example 6 in mice treated with fluoropyrimidines

The experiment shown in Table 2 demonstrates that compound A of Example 6 increases 5-FU AUC (Area under curve) selectively in tumors in mice treated with fluoropyrimidines. In this study, fluoropyrimidines, such as 5-FU, doxifluridine [5'-deoxy-5-fluorouridine] and capecitabine [5'-deoxy-5-fluoro-N⁴-(n-pentyloxycarbonyl)cytidine], were given to BALB/c nude mice bearing the human gastric cancer xenograft MKN28 in combination with either compound A or 5-EU. Then, 5-FU levels in the plasma and tumor tissues were measured at 0.25, 0.5, 2, 4 and 7 hours after each fluoropyrimidine administration (n = 3 mice), and 5-FU AUC was calculated. The known DPD inhibitor 5-EU greatly increased the 5-FU AUCs both in the plasma and tumor tissues in mice treated with either 5-FU, capecitabine or doxifluridine . Since the increase of 5-FU levels in the plasma results in the systemic toxicity of 5-FU, 5-EU should enhance both the efficacy and toxicity of the fluoropyrimidines.
In contrast, compound A greatly increases the 5-FU AUCs only in tumor tissues probably as a result of compound A's tumor selective inhibition of DPD activity that catabolizes 5-FU. Compound A of Example 6 therefore enhances the efficacy of fluoropyrimidines with little increasing their toxicity.

**Table 2. 5-FU AUC in the plasma and tumors in mice treated with fluoropyrimidines**

| Test compounds (µmol/kg) | Fluoropyrimidines (mmol/kg) | 5-FU AUC (nmolohr/mL) | |
|---|---|---|---|
| | | Plasma | Tumor |
| Exp. 1 | | | |
| - | 5-FU (0.3) | 9.3 | 1.3 |
| Compound A (2) | 5-FU (0.3) | 9.5 | 6.0 |
| 5-EU (1) | 5-FU (0.3) | 75 | 48 |
| - | Capecitabine (1.5) | 1.3 | 30 |
| Compound A (2) | Capecitabine (1.5) | 3.1 | 67 |
| 5-EU (1) | Capecitabine (1.5) | 53 | 120 |

| Exp. 2 | | | |
|---|---|---|---|
| - | Doxifluridine (0.75) | 2.6 | 8.0 |
| Compound A (2) | Doxifluridine (0.75) | 11 | 30 |
| 5-EU (1) | Doxifluridine (0.75) | 86 | 73 |
| - | Capecitabine (1.5) | 1.5 | 30 |
| Compound A (2) | Capecitabine (1.5) | 3.8 | 76 |
| 5-EU (1) | Capecitabine (1.5) | 54 | 120 |

### 3. Enhancement of antitumor activity of Capecitabine by Compound A of Example 6

Compound A of Example 6 was examined for its activity to enhance the efficacy of capecitabine in BALB/c nude mice bearing the human prostate cancer xenograft PC-3. Compound A and capecitabine were orally given simultaneously or sequentially 5 successive days per week for 3 weeks starting on day 53 after the tumor inoculation when the tumor becomes palpable. On day 75, tumor volume gain and the percentage of the tumor growth inhibition were calculated. As Table 3 shows, capecitabine inhibited tumor growth to a greater extent when compound A was given in combination either simultaneously or sequentially. Since compound A itself is not cytotoxic (data not shown), it enhances the efficacy of capecitabine by inhibiting DPD activity.

**Table 3 Enhancement of Capecitabine Efficacy by compound A of Example 6**

| Capecitabine (mmol/kg/d) | Compound A (µmol/kg/d) | Tumor volume change (mm³) Day 53-75 | Tumor growth inhibition (%) Day 75 | Body weight change (g) Day 75 | Survivors on Day 75 |
|---|---|---|---|---|---|
| - | - | 981 | - | -3.6 | 5/5 |
| 1.0 | - | 757 | 23 | -3.4 | 5/5 |
| 1.0 | 1.0 | 323* | 67 | -1.8 | 5/5 |
| 1.0 | 1.0^{#} | 201* | 80 | -0.3 | 4/5 |

| | | | | | |
|---|---|---|---|---|---|
| * p < 0.05 as compared with the control group # Compound A was given one hour prior to the capecitabine administration. | | | | | |

The following Examples are intended to illustrate the present invention in more detail, but are not intended to limit its scope in any manner.

### Reference example 1:

### a) Preparation of 2',3'-di-O-acetyl-5'-deoxy-5-ethynyluridine

5-Ethynyluracil (12g, 88.2 mmol) was suspended in a solution of ammonium sulfate (570mg, 4.3 mmol) in hexamethyldisilazane (240ml). The suspension was refluxed for 6hr. After concentrating the reaction mixture under reduced pressure, a solution of 5-deoxy-1,2,3-tri-O-acetyl-D-ribofranoside (27.5g, 105.8 mmol) in acetonitrile (300ml) was added to the residue. Then, a solution of anhydrous stannic tetrachloride (27.6g, 105.8 mmol) in nitromethane (60ml) was added dropwise to the mixture with keeping the temperature below 0°C. After stirring the mixture at 0°C for additional 4hr, sodium bicarbonate was added and followed by dropwise addition of water. After the mixture was stirred for 2hr, the reaction mixture was filtered to remove insoluble material, which was washed with ethyl acetate.The filtrate and washing were combined and dried over MgSO₄ and filtered. The filtrate was evaporated under reduced pressure.
Purification of the residue by silica gel chromatography ( using n-hexane : ethyl acetate= 1:2 as an eluent) gave 2',3'-di-O-acetyl-5'-deoxy-5-ethynyluridine (13.7g, 48%yield ).
MALDI-MS: (m/z) 359[M+Na]⁺, 375[M+K]⁺
¹H-NMR : (270MHz;CDCl₃) : δ 1.47 (3H, d, J = 6.6), 2.10 (3H, s), 2.12 (3H, s), 3.23 (1H, s), 4.19-4.28 (1H, m), 5.01-5.05 (1H, m), 5.30-5.34 (1H, m), 5.90 (1H, d, J = 4.95), 7.57 (1H, s), 8.34 (1H, br.s)

### b) Preparation of 2',3'-bis-O-(tert-butyldimethylsilyl)-5'-deoxy-5-ethynyluridine

To a solution of 2',3'-di-O-acetyl-5'-deoxy-5-ethynyluridine (13.7g, 40.7 mmol) was dissolved in methanol (100ml) was added dropwise a solution of sodium hydroxide (3.3g, 81.4 mmol) in water (10ml) with stirring at 0°C. After stirring at 0°C for additional 30min, the reaction mixture was adjusted to pH7 with aqueous 1N-hydrochloric acid. Then the mixture was evaporated under reduced pressure.
The residue was dissolved in DMF (250ml), and imidazole (41.6g, 610 mM) and tert-butyldimethylchlorosilane (30.7g, 203 mmol) was added to the solution with stirring. The mixture was continued to stir for 23hr. The reaction mixture was partitioned between ethyl acetate and water. The aqueous layer was back-extracted with ethyl acetate. The combined organic layers were washed with brine, dried over Na₂SO₄, filtered and evaporated under reduced pressure. Purification of the residue by silica gel chromatography ( using n-hexane : ethyl acetate = 3:1 as an eluent) gave 2',3'-bis-O-(tert-butyldimethylsilyl)-5'-deoxy-5-ethynyluridine (14.9g, 76%yield).
FAB-MS : (m/z) 481[M+H]⁺
1H-NMR : (270MHz;CDCl₃) : δ 0.10-0.13 (12H, m), 0.91 (18H, m), 1.40 (3H, d, J = 6.6), 3.21(1H, s), 3.58 (1H, dd, J = 4.29, 6.6), 4.08-4.17 (2H, m), 5.62(1H, d, J = 2.64), 7.68(1H, s), 8.24(1H, br.s)

The following compounds were prepared in the same manner as described above using the corresponding known 5-substituted uracil derivatives:

### 2',3'-bis-O-(tert-butyldimethylsilyl)-5'-deoxy-5-iodouridine,

FAB-MS : (m/z) 583[M+H]⁺, 605[M+Na]⁺
¹H-NMR : (270MHz;DMSO-d₆) : δ -0.09 (3H, s), -0.03 (3H, s), 0.00 (3H, s), 0.02 (3H, s), 0.75 (9H,s), 0.81 (9H,s), 1.24 (3H, d, J = 6.6), 3.75 (1H,dd, J = 4.6, 4.0), 3.86 (1H, m), 4.36 (1H, dd, J = 5.3, 5.0), 5.59 (1H, d, J = 5.6), 7.91 (1H, s), 11.69 (1H, br.s)

### 2',3'-bis-O-(tert-butyldimethylsilyl)-5'-deoxy-5-trifluoromethyluridine,

FAB-MS : (m/z) 525 [M+H]⁺
¹H-NMR : (400MHz;CDCl₃) : δ 0.00 (6H, s), 0.02 (3H, s), 0.06 (3H, s), 0.83 (9H, s), 0.83 (9H, s), 1.32 (3H, d, J = 5.9), 3.47 (1H, m), 4.05 (1H, m), 4.16 (1H, m), 5.54 (1H, d, J = 2.2), 7.84 (1H, s), 8.43 (1H, br.s)

### 2',3'-bis-O-(tert-butyldimethylsilyl)-5-(3-benzyloxybenzyl)-5'-deoxy-uridine,

FAB-MS : (m/z) 653 [M+H]⁺
¹H-NMR : (270MHz;CDCl₃) : δ -0.09-0.01 (12H, m), 0.77-0.82 (18H, m), 0.90 (3H, d, J = 6.3), 3.27 (1H, m), 3.31 (1H, d, J = 16.5), 3.61 (1H, d, J = 16.5), 3.86 (1H, m), 3.95 (1H, m), 4.94 (2H, s), 5.50 (1H, d, J = 2.0), 6.68-6.78 (4H, m), 7.12-7.34 (6H, m), 8.54 (1H, br.s)

The following compounds can be prepared in the same manner as described above using the corresponding known 5-substituted uracil derivatives:
2',3'-bis-O-(tert-butyldimethylsilyl)-5'-deoxy-5-prop-1-ynyluridine,
2',3'-bis-O-(tert-butyldimethylsilyl)-5-but-1-ynyl-5'-deoxyuridine,
2',3'-bis-O-(tert-butyldimethylsilyl)-5'-deoxy-5-pent-1-ynyluridine,
2',3'-bis-O-(tert-butyldimethylsilyl)-5'-deoxy-5-hex-1-ynyluridine,
5-bromo-2',3'-bis-O-(tert-butyldimethylsilyl)-5'-deoxyuridine,
2',3'-bis-O-(tert-butyldimethylsilyl)-5-(1-chlorovinyl)-5'-deoxyuridine,
2',3'-bis-O-(tert-butyldimethylsilyl)-5'-deoxy-5-vinyluridine,

### Example 1:

### Preparation of 2',3'-bis-O-(tert-butyldimethylsilyl)-5'-deoxy-5-ethynylcytidine

To a solution of dimethylaminopyridine (19.0g, 155.5 mmol) in acetonitrile (120 ml) and pyridine (12.6ml, 155.5 mmol) phosphoryl chloride (14.4g, 93.8mM) was added dropwise in an ice bath under Ar atmosphere. After stirring the mixture for 1h at room temperature, a solution of 2',3'-bis-O-(tert-butyldimethylsilyl)-5'-deoxy-5-ethynyluridine (14.9g, 31.1 mmol) in acetonitrile (80 ml) was added at 5°C with cooling in an ice bath. The mixture was stirred at room temperature for 2h. Then, 25% aq. ammonia solution (10 ml) was added in one portion to the reaction mixture while keeping the temperature below 10°C. A second portion of 25 % aq. ammonia solution (65ml) was added to the reaction mixture while keeping the temperature below 10°C. The mixture was stirred at room temperature for 45 min. Then the reaction mixture was diluted with water (200ml) at room temperature and extracted three times with ethyl acetate. The combined organic layers were washed successively with aq. 1N-hydrochloric acid solution, aq. saturated sodium bicarbonate and brine. The organic layer was dried over MgSO₄, filtrered and evaporated under reduced pressure. Purification of the residue by silica gel chromatography (using n-hexane : ethyl acetate = 2 : 1 as an eluent ) gave 2',3'-bis-O-(tert-butyldimethylsilyl)-5'-deoxy-5-ethynylcytidine (14.8g, 99% yield).
MALDI-MS : (m/z) 502[M+Na]⁺, 518[M+K]⁺
¹H-NMR : (400MHz;CDCl₃) : δ 0.05 (3H, s), 0.06 (3H, s), 0.12 (3H, s), 0.24 (3H, s), 0.89 (9H, s), 0.92 (9H, s), 1.41 (3H, d, J = 6.35), 3.36 (1H, s), 3.46 (1H, dd, J = 3.91,7.81), 4.19-4.26 (2H, m), 5.57 (1H, s), 5.79 (1H, br.s), 7.57 (1H, br.s), 7.80(1H, s)

The following compounds were obtained in a manner analogous to that of Example 1.

### Example 2: 2',3'-bis-O-(tert-butyldimethylsilyl)-5'-deoxy-5-iodocytidine,

FAB-MS : (m/z) 582[M+H]⁺
¹H-NMR : (270MHz;DMSO-d6) δ 0.00 (3H, s), 0.02 (3H, s), 0.06 (3H, s), 0.08 (3H, s), 0.82 (9H, s), 0.88 (9H, s), 1.30 (3H, d, J = 6.6), 3.78 (1H, dd, J = 4.6, 4.3), 3.93 (1H, m), 4.33 (1H, dd, J = 4.9, 4.6), 5.67 (1H, d, J = 5.0), 6.67(1H, br.s), 7.87(2H, br.s)

### Example 3: 2',3'-bis-O-(tert-butyldimethylsilyl)-5'-deoxy-5-trifluoro-methylcytidine,

FAB-MS : (m/z) 524 [M+H]⁺
¹H-NMR : (400MHz;CDCl₃) : δ 0.00 (6H, s), 0.08 (3H, s), 0.19 (3H, s), 0.84 (9H, s), 0.87 (9H, s), 1.35 (3H, d, J = 6.6), 3.38 (1H, m), 4.15 (1H, m), 4.21 (1H, m), 5.51 (1H, s), 7.97 (1H, s)

### Example 4: 5-(3-benzyloxybenzyl)-2',3'-bis-O-(tert-butyldimethylsilyl)-5'-deoxycytidine,

FAB-MS : (m/z) 652 [M+H]⁺
¹H-NMR : (270MHz;CDCl₃) : δ -0.01 (3H, s), 0.00 (3H, s), 0.09 (3H, s), 0.22 (3H, s), 0.86 (9H, s), 0.90 (9H, s), 1.10 (3H, d, J = 6.6), 3.37 (1H, m), 3.57 (2H, s), 4.08-4.18 (2H, m), 5.03 (2H, s), 5.59 (1H, s), 6.75-6.90 (3H, m), 7.11 (1H, s), 7.26 (1H, m), 7.31-7.44 (5H, m)

### Example 5: 2',3'-bis-O-(tert-butyldimethylsilyl)-5-cyano-5'-deoxycytidine

FAB-MS : (m/z) 481[M+H]⁺
¹H-NMR : (270MHz;DMSO-d₆) : δ -0.04 (3H, s), 0.00 (3H, s), 0.02 (3H,s), 0.76 (9H, s), 0.82 (9H, s), 1.21 (3H, d, J = 6.3), 3.81 (1H, m), 4.05 (1H, t, J = 5.0), 4.71 (1H, t, J = 5.0), 5.65 (1H, d, J = 5.3), 6.41 (1H, s), 7.69 (1H, br.s), 7.85 (1H, br.s)

The following compounds can be obtained according to a manner analogous to that of Example 5.
2',3'-bis-O-(tert-butyldimethylsilyl)-5'-deoxy-5-prop-1-ynylcytidine,
2',3'-bis-O-(tert-butyldimethylsilyl)-5-but-1-ynyl-5'-deoxycytidine,
2',3'-bis-O-(tert-butyldimethylsilyl)-5'-deoxy-5-pent-1-ynylcytidine,
2',3'-bis-O-(tert-butyldimethylsilyl)-5'-deoxy-5-hex-1-ynylcytidine,
5-bromo-2',3'-bis-O-(tert-butyldimethylsilyl)-5'-deoxycytidine,
2',3'-bis-O-(tert-butyldimethylsilyl)-5-(1-chlorovinyl)-5'-deoxycytidine,
2',3'-bis-O-(tert-butyldimethylsilyl)-5'-deoxy-5-vinylcytidine,

### Example 6:

### Preparation of 5'-deoxy-5-ethynyl-N⁴-(n-pentyloxycarbonyl)cytidine,

a) 2',3'-Bis-O-(tert-butyldimethylsilyl)-5'-deoxy-5-ethynylcytidine (45 mg, 0.09 mmol) was dissolved in dichloromethane (1ml) and pyridine (33µl,0.42mM). To the mixture, n-pentyl chloroformate (42mg, 0.28 mmol) was added dropwise in an ice bath under Ar. The reaction mixture was stirred at room temperature for 2h. Water was aded and the reaction mixture stirred for 30 mins. The reaction mixture was partitioned between dichloromethane and water. The aqueous layer was extracted with dichloromethane. The combined organic layers were dried over Na₂SO₄ and filtered. The filtrate was evaporated under reduced pressure.
   Purification of the residue by silica gel chromatography (using n-hexane : ethyl acetate = 4 : 1 as an eluent ) gave 2',3'-bis-O-(tert-butyldimethylsilyl)-5'-deoxy-5-ethynyl-N⁴-(n-pentyloxycarbonyl)cytidine (40 mg, 72% yield).
   FAB-MS : (m/z) 594[M+H]⁺
   ¹H-NMR : (270MHz;CDCl₃) : δ 0.12-0.27 (12H, m), 0.90-0.92 (21H, m), 1.26-1.42 (7H, m), 1.64-1.74 (2H, m), 3.25-3.51 (2H, m), 4.15-4.23 (4H, m), 5.55-5.60 (1H, m), 7.62 (0.5H, br.s), 7.73 (0.5H, br.s), 8.00 (0.5H, br.s), 12.3 (0.5H, br.s)
b) To a solution of 2',3'-bis-O-(tert-butyldimethylsilyl)-5'-deoxy-5-ethynyl-N⁴-(n-pentyloxycarbonyl)cytidine (19mg, 0.03 mmol) in tetrahydrofuran (500µl) was added dropwise tetrabutylammonium fluoride (93µl, 0.09 mmol) [1.0M tetrahydrofuran solution] at room temperature under Ar atmosphere. After the mixture was stirred at room temperature for 2h., the reaction mixture was evaporated under reduced pressure. The residue was partitioned between dichloromethane and water. The aqueous layer was back-extracted with dichloromethane. The combined organic layers were dried over Na₂SO₄, filtered and evaporated under reduced pressure. Purification of the residue by silica gel chromatography (using dichloromethane : methanol = 20 : 1 as an eluent) gave 5'-deoxy-5-ethynyl-N⁴-(n-pentyloxycarbonyl)cytidine (compound A) (9 mg, 81% yield).
   FAB-MS : (m/z) 366[M+H]⁺
   ¹H-NMR : (400MHz;DMSO-d₆) : δ 0.88 (3H, t, J=6.84), 1.30-1.32 (7H, m), 1.59-1.63 (2H, m), 3.67-3.71 (1H, m), 3.90-4.46 (5H, m), 5.07 (1H, m), 5.42 (1H, m), 5.66 (1H, m), 7.89 (0.5H, br.s), 8.14 (0.5H, br.s), 9.53 (0.5H, br.s), 11.7 (0.5H, br.s)

The following compounds (examples 7-41) were obtained in a manner analogous to that of Example 6.

### Example 7: 5'-deoxy-5-ethyl-N⁴-(n-pentyloxycarbonyl)cytidine

FAB-MS : (m/z) 370[M+H]⁺
¹H-NMR : (270MHz;CDCl₃) : δ 0.91(3H, t, J = 6.93), 1.16 (3H, t, J = 7.5), 1.36 (4H, m), 1.41 (3H, d, J = 6.6), 1.72 (2H, m), 2.47 (2H, q, J = 7.5), 3.22 (1H, br.s), 3.93 (1H, m), 4.16 (2H, t, J = 6.93), 4.28 (2H, m), 4.49 (1H, br.s), 5.66 (1H, d, J = 3.63), 7.37 (1H, br.s), 12.46(1H, br.s)

### Example 8: 5'-deoxy-5-iodo-N⁴-(n-pentyloxycarbonyl)cytidine

FAB-MS : (m/z) 468 [M+H]⁺, 490[M+Na]⁺
¹H-NMR : (270 MHz; DMSO-d₆) : δ 1.36 (3H, t, J = 7.0), 1.76-1.78 (7H, m), 2.09 (2H, m), 4.18 (1H, m), 4.36 (1H, m), 4.54 (2H, t, J = 5.9), 5.54 (1H, br.d, J = 5.0), 5.84 (1H, br.d, J = 5.0), 6.09 (1H, d, J = 4.3), 8.47 (1H, s), 12.24 (1H, br.s)

### Example 9: 5'-deoxy-N⁴-(n-pentyloxycarbonyl)-5-trifluoromethylcytidine

FAB-MS : (m/z) 410[M+H]⁺
¹H-NMR : (270MHz;CDCl₃) : δ 0.88-0.94 (3H, m), 1.32-1.39 (4H, m), 1.42 (3H, d, J = 6.6), 1.68-1.75 (2H, m), 3.09-3.30 (1H, m), 3.92 (1H, m), 4.15-4.27 (5H, m), 5.67 (1H, d, J = 3.3), 8.05-8.31 (1H, m), 12.6 (1H, br.s)

### Example 10: 5-(3-benzyloxybenzyl)-5'-deoxy-N⁴-(n-pentyloxy-carbonyl)cytidine

FAB-MS : (m/z) 538 [M+H]⁺
¹H-NMR : (270MHz;CDCl₃) : δ 0.90 (3H, t, J = 6.9), 1.04 (3H, d, J = 6.6), 1.26-1.39 (4H, m), 1.72 (2H, m), 3.16 (1H, br.s), 3.67 (1H, d, J = 16.5), 3.71 (1H, m),3.75 (1H, d, J = 16.5), 4.10 (2H, m), 4.16 (2H, t, J = 6.9), 4.40 (1H, br.s), 5.04 (2H, s), 5.62 (1H, d, J = 3.3), 6.79 (1H, d, J = 7.6), 6.84-6.89 (2H, m), 6.97 (1H, br.s), 7.22-7.43 (6H, m), 12.41 (1H, br.s)

### Example 11: 5-cyano-5'-deoxy-N⁴-(n-pentyloxycarbonyl)cytidine

FAB-MS : (m/z) 367[M+H]⁺
¹H-NMR : (270MHz;DMSO-d₆) : δ 0.88 (3H, t, J = 6.9), 1.30 (4H, s), 1.31 (3H, d, J = 6.3), 1.62 (2H, m), 3.81 (1H, quin., J = 6.3), 3.91 (1H, quin., J = 6.3), 4.13 (2H, t, J = 6.6) , 4.39 (1H, m), 5.09 (1H, d, J = 6.3), 5.31 (1H, d, J = 5.3), 5.83( 1H, d, J = 4.0), 7.57 (1H, s), 11.23 (1H, br.s)

### Example 12: 5'-deoxy-5-ethynyl-N⁴-(n-propoxycarbonyl)cytidine

FAB-MS : (m/z) 338[M+H]⁺, 360[M+Na]⁺
¹H-NMR : (270MHz;DMSO-d6) : δ 0.91 (3H, t, J = 7.3), 1.31 (3H, d, J = 6.3), 1.63 (2H, sextet, J = 7.3), 3.69 (1H, dt, J = 5.9,5.3), 3.91 (1H, quin., J = 5.9), 4.03 (2H, t, J = 6.6), 4.13 (1H, dt, J = 5.0,4.3), 4.35 (1H, br.s), 5.05 (1H, d, J = 5.9), 5.41 (1H, d, J = 5.3), 5.66 (1H, d, J = 4.0), 8.01 (1H, br.s)

### Example 13: 5'-deoxy-5-ethynyl-N⁴-(isopropoxycarbonyl)cytidine

FAB-MS : (m/z) 338[M+H]⁺
¹H-NMR : (270MHz;DMSO-d6) : δ 1.24 (6H, d, J = 5.9), 1.31 (3H, d, J = 6.6), 3.68 (1H, dt, J = 5.9,5.6), 3.90 (1H, quin., J = 5.9), 4.12 (1H, m), 4.30 (1H, s), 4.85 (1H, m), 5.05 (1H, d, J = 5.9), 5.40 (1H, d, J = 5.3), 5.66 (1H, d, J = 3.6), 8.02 (1H, br.s)

### Example 14: N⁴-(isobutoxycarbonyl)-5'-deoxy-5-ethynylcytidine

FAB-MS : (m/z) 352[M+H]⁺
¹H-NMR : (270MHz;DMSO-d6) : δ 0.91 (6H, d, J = 6.6), 1.30 (3H, d, J = 6.3), 1.91 (2H, m), 3.68 (1H, dt, J = 5.9,5.3), 3.84 (2H, d, J = 6.6), 3.89 (1H, quin., 6.3), 4.11 (1H, m), 4.30 (1H, s), 5.03 (1H, d, J = 5.9), 5.38 (1H, d, J = 5.3), 5.66 (1H, d, J = 4.0), 7.96(1H, s)

### Example 15: 5'-deoxy-5-ethynyl-N⁴-[(2-methylpentyloxy)carbonyl]-cytidine

FAB-MS : (m/z) 380[M+H]⁺,402[M+Na]⁺
¹H-NMR : (270MHz;DMSO-d6) : δ 0.85-0.93 (7H, m), 1.31 (3H, d, J = 6.3), 1.28-1.37 (3H, m), 1.77 (1H, m), 3.69 (1H, dt, J = 5.9, 5.6), 3.88(2H, m), 3.92(1H, m), 4.13 (1H, dt, J = 4.9, 4.6), 4.37(1H, br.s), 5.06 (1H, d, J = 5.9), 5.41 (1H, d, J = 5.3), 5.66 (1H,d,J = 4.0), 8.02 (1H, br.s),

### Example 16: 5'-deoxy-5-ethynyl-N⁴-[(3-methylpentyloxy)carbonyl]-cytidine

FAB-MS : (m/z) 380[M+H]⁺
¹H-NMR : (270MHz;CDCl₃) : δ 0.86-0.98 (6H, m), 1.15-1.80 (8H, m), 3.25-3.26 (1H, m), 3.53 (1H, brs), 3.90-3.95 (1H, m), 4.25-4.37 (4H, m), 5.33 (1H, brs), 5.71 (1H, d, J = 4.28), 7.69 (1H, br.s), 8.13 (1H, br.s),

### Example 17: 5'-deoxy-5-ethynyl-N⁴-[(2-propylpentyloxy)carbonyl]-cytidine

MALDI-MS : (m/z) 408.5[M+H]⁺, 430.5[M+Na]⁺, 446[M+K]⁺
¹H-NMR : (270MHz;DMSO-d6) : δ 0.87 (6H, br.m), 1.29 (11H, br.m),1.66 (1H, br.m), 3.69 (1H, br.m), 3.94-4.5 (5H, br.m), 5.06 (1H, br.m), 5.42 (1H, br.m), 5.66 (1H, br.m), 7.90 (0.5H, br.s), 8.14 (0.5H, br.s), 9 .53 (0.5H, br.s)

### Example 18: 5'-deoxy-5-ethynyl-N⁴-(n-octyloxycarbonyl)cytidine

FAB-MS : (m/z) 408[M+H]⁺, 430[M+Na]⁺
¹H-NMR : (270MHz;DMSO-d6) : δ 0.86 (3H, t, J = 5.0), 1.26 (10H, m), 1.31 (3H, d, J = 6.0), 1.60 (2H, m), 3.69 (1H, dt, J = 5.9,5.6), 3.90 (1H, quin., J = 6.3), 4.06 (2H, t, J = 6.3), 4.13 (1H, m), 4.35 (1H, br.s), 5.05 (1H, d, J = 5.9), 5.41 (1H, d, J = 5.3), 5.66 (1H, d, J = 4.0), 8.02 (1H, br.s)

### Example 19: 5'-deoxy-N⁴-[(2-ethylhexyl)oxycarbonyl]-5-ethynyl-cytidine

FAB-MS : (m/z) 408[M+H]⁺
¹H-NMR : (270MHz;CDCl₃) : δ 0.88-0.94 (6H, m), 1.30-1.41 (12H, m), 3.25 (1H, d, J = 3.63), 3.53 (1H, m), 3.92-3.94 (1H, m), 4.15-4.37 (4H, m), 5.32 (1H, m), 5.70 (1H, dt, J = 4.61), 7.86 (1H, br.s), 8.14 (1H, br.s)

### Example 20: 5'-deoxy-5-ethynyl-N⁴-[(2-phenylethoxy)carbonyl]-cytidine

FAB-MS : (m/z) 400[M+H]⁺
¹H-NMR : (270MHz;DMSO-d6) : δ 1.31 (3H, d, J = 6.3), 2.94 (2H, t, J = 6.9), 3.69 (1H, dt, J = 5.9,5.6), 3.90 (1H, quin., J = 6.3), 4.14 (1H, m), 4.28 (2H, t, J = 6.9), 4.31 (1H, br.s), 5.05 (1H, d, J = 5.9), 5.41 (1H, d, J = 4.9), 5.66 (1H, d, J = 4.0), 7.27 (5H, m), 8.01 (1H, br.s),

### Example 21: N⁴-(cyclohexyloxycarbonyl)-5'-deoxy-5-ethynylcytidine

FAB-MS : (m/z) 378[M+H]⁺
¹H-NMR : (270MHz;DMSO-d6) : δ 1.06-1.48 (9H, m), 1.69 (2H, m), 1.86 (2H, m), 3.65-3.72 (1H, m), 3.88-3.93 (1H ,m), 4.13-4.61 (3H, m), 5.06 (1H, d, J = 6.27), 5.42 (1H, d, J = 4.95), 5.66 (1H, d, J = 3.63), 7.9-8.1 (1H, m), 9.4 (0.5H, br.s), 11.8(0.5H, br.s)

### Example 22: N⁴-[(cyclohexylmethoxy)carbonyl]-5'-deoxy-5-ethynylcytidine

FAB-MS : (m/z) 392[M+H]⁺, 414[M+Na]⁺
¹H-NMR : (270MHz;DMSO-d6) : δ 0.86-1.25 (5H, m), 1.31 (3H, d, J = 6.3), 1.61-1.72 (6H, m), 3.69 (1H, dt, J = 5.9,5.6), 3.89 (2H, d, J = 6.3), 3.90 (1H, m), 4.14 (1H, m), 4.36 (1H, br.s), 5.05 (1H, d, J = 5.9), 5.41 (1H, d, J = 5.3), 5.66 (1H, d, J = 4.0), 8.02 (1H, br.s).

### Example 23: 5'-deoxy-5-ethynyl-N4-(neopentyloxycarbonyl)-cytidine

FAB-MS : (m/z) 366[M+H]⁺, 388[M+Na]⁺
¹H-NMR : (270MHz;DMSO-d6) : δ 0.93 (9H, s), 1.30 (3H, br.d), 3.67-4.27 (5.5H, br.m), 4.47 (0.5H, br.s), 5.06 (1H, br.m), 5.39 (1H, br.m), 5.43 (1H, br.m), 7.88 (0.5H, br.s), 8.16 (0.5H, br.s), 9.56 (0.5H, br.s), 11.69 (0.5H, br.s)

### Example 24: 5'-deoxy-N4-[(3,3-dimethylbutoxy)carbonyl]-5-ethynylcytidine

FAB-MS : (m/z) 380[M+H]⁺
¹H-NMR : (270MHz;DMSO-d6) : δ 1.01 (9H, s), 1.39 (3H, br.d), 1.63 (2H, br.t), 3.77 (1H, br.m), 3.98-4.32 (4.5H, br.m), 4.56 (0.5H, br.s), 5.13 (1H, br.m), 5.45-5.51 (1H, br.m), 5.73-5.75 (1H, br.m), 7.96 (0.5H, br.s), 8.23 (0.5H, br.s), 9.57 (0.5H, br.s), 11.76 (0.5H, br.s)

### Example 25: 5'-deoxy-5-ethynyl-N4-(tridecyloxycarbonyl)cytidine

MALDI-MS : (m/z) 478[M+H]⁺, 516[M+K]⁺
¹H-NMR : (270MHz;DMSO-d6) : δ 0.85(3H, d, J = 4.6), 1.24(20H, m), 1.30 (3H, d, J = 6.3), 1.60 (2H, m), 3.68 (1H, dt, J = 5.9,5.6), 3.90 (1H, quin., J = 6.3), 4.05 (2H, t, J = 6.6), 4.13 (1H, dt, J = 5.0,4.3), 4.34 (1H, br.s), 5.05 (1H, d, J = 5.9), 5.40 (1H, d, J = 5.3), 5.65 (1H, d, J = 3.6), 8.00 (1H, br.s)

### Example 26: N⁴-(n-butoxycarbonyl)-5'-deoxy-5-ethynylcytidine

FAB-MS : (m/z) 352 [M+H]⁺, 374 [M+Na]⁺
¹H-NMR : (270 MHz; DMSO-d₆) : δ 0.89 (3H, t, J = 7.2), 1.28 - 1.41 (5H, m), 1.53 - 1.64 (2H, m), 3.64 -3.71 (1H, m), 3.85 - 3.92 (1H, m), 4.03 - 4.15 (3H, m), 4.34 (1H, s), 5.04 (1H, d, J = 5.9), 5.39 (1H, d, J = 5.3), 5.64 (1H, d, J = 3.6), 8.06 (1H, br.s)

### Example 27: 5'-deoxy-5-ethynyl-N⁴-(n-hexyloxycarbonyl)cytidine

FAB-MS : (m/z) 380 [M+H]⁺, 402 [M+Na]⁺
¹H-NMR : (270 MHz; DMSO-d₆) : δ 0.95 (3H, t, J = 6.6), 1.38 - 1.40 (9H, m), 1.63 - 1.71 (2H, m), 3.74 -3.80 (1H, m), 3.94 - 4.03 (1H, m), 4.14 (2H, t, J = 6.6), 4.19 - 4.24 (1H, m), 4.43 (1H, s), 5.13 (1H, d, J = 5.9), 5.49 (1H, d, J = 5.3), 5.74 (1H, d, J = 4.0), 8.09 (1H, br.s)

### Example 28: 5'-deoxy-5-ethynyl-N⁴-(n-decyloxycarbonyl)cytidine

MS:FAB-MS : (m/z) 436 [M+H]⁺
¹H-NMR : (270MHz; DMSO-d6) : d 0.85 (3H, t, J = 6.4), 1.15 - 1.42 (17H, m), 1.60 (2H, m), 3.69 (1H, m), 3.90 (1H, m), 4.05 (2H, t, J = 6.6), 4.13 (1H, m), 4.34 (1H, br.s), 5.04 (1H, d, J = 5.6), 5.40 (1H, d, J = 4.9), 5.66 (1H, d, J = 3.6), 8.01 (1H, br.s)

### Example 29: 5'-deoxy-5-ethynyl-N⁴-[(2,6-dimethylcylohexyloxy)-carbonyl]cytidine

MS:FAB-MS : (m/z) 406 [M+H]+
¹H-NMR : (270MHz; DMSO-d6) : d 0.83 (36H, d, J = 6.3), 1.20 - 1.50 (9H, m), 1.55 - 1.75 (2H, m), 3.68 (1H, m), 3.93 (1H, m) 4.12 - 4.20 (2H, m), 4.45 (0.7H, s), 4.86 (0.3H, s), 5.04 (1H, d, J = 5.6), 5.43 (1H, br.s), 5.67 (1H, br.s), 7.96 (0.3H, br.s), 8.14 (0.7H, br.s), 9.50 (0.7H, br.s) 12.00 (0.3H, br.s)

### Example 30: 5'-deoxy-5-ethynyl-N⁴-(benzyloxycarbonyl)cytidine

MS:FAB-MS : (m/z) 386 [M+H]+
¹H-NMR : (270MHz; DMSO-d6) : d 1.30 (3H, d, J = 6.3), 3.69 (1H, m), 3.89 (1H, m), 4.13 (1H, m), 4.35 (1H, br.s), 5.05 (1H, d, J = 5.9), 5.14 (2H, s), 5,41 (1H, d, J = 5.3), 5.66 (1H, d, J = 3.6), 7.31 - 7.45 (5H, m), 8.01 (1H, br.s)

### Example 31: 5'-deoxy-5-ethynyl-N⁴-[(1-isopropyl-2-methylpropoxy)-carbonyl]cytidine

MS:FAB-MS : (m/z) 394 [M+H]+
¹H-NMR : (270MHz; DMSO-d6) : d 0.93 (12H, d, J = 6.6), 1.40 (3H, d, J = 6.6), 1.97 (2H, m), 3.33 (1H, d, J = 3.6), 3.55 (1H, s), 3.91 (1H, m), 4.30 (1H, m), 4.36 (1H, m), 4.62 (1H, m), 5.40 (1H, s), 5.72 (1H, d, J = 4.3), 7.69 (1H, s), 8.11 (1H,s)

### Example 32: 5'-deoxy-5-ethynyl-N⁴-[(3-methylbenzyloxy)-carbonyl]cytidine

MS:FAB-MS : (m/z) 416 [M+H]+
¹H-NMR : (270MHz; DMSO-d6) : d 1.31 (3H, d, J = 6.0), 3.70 (1H, m), 3.76 (3H, s), 3.90 (1H, m) 4.14 (1H, m), 4.26 (0.5H, br.s), 4.44 (0.5H, br.s), 5.06 (2H, s), 5.16 (1H, br.s), 5.41 (1H, br.s), 5.66 (1H, m), 6.91 (1H, d, J = 7.9), 7.00 (2H, m), 7.30 (1H, dd, J = 7.9, 7.9), 7.89 (0.5H, br.s), 8.14 (0.5H, br.s), 9.72 (0.5H, br.s), 11.7 (0.5H, br.s)

### Example 33: 5'-deoxy-5-ethynyl-N⁴-(methoxycarbonyl)cytidine

MS:FAB-MS : (m/z) 310 [M+H]+
¹H-NMR : (270MHz; DMSO-d6) : d 1.30 (3H, d, J = 6.3), 3.66 (3H, s), 3.70 (1H, m), 3.90 (1H, quin., J = 6.3), 4.13 (1H, m), 4.34 (1H, s), 5.05 (1H, d, J = 5.9), 5.40 (1H, d, J = 5.3) 5.66 (1H, d, J = 4.0), 8.00 (1H, br.s)

### Example 34: 5'-deoxy-5-ethynyl-N⁴-(ethyloxycarbonyl)cytidine

MS:FAB-MS : (m/z) 324 [M+H]+
¹H-NMR : (270MHz; DMSO-d6) : d 1.23 (3H, t, J = 6.93), 1.31 (3H, d, J = 6.27), 3.69 (1H, m), 3.90 (1H, m), 4.08 - 4.14 (3H, m), 4.35 (1H, br.s), 5.05 (1H, d, J = 5.94), 5.40 (1H, d, J = 5.27), 5.66 (1H, d, J = 3.63), 8.02 (1H, br.s)

### Example 35: 5'-deoxy-N⁴-(n-pentyloxycarbonyl)cytidine

FAB-MS : (m/z) 342[M+H]⁺,
¹H-NMR : (270MHz;DMSO-d6) : δ 0.88 (3H, t, J=6.9), 1.31(4H, m), 1.32 (3H, d, J=6.3), 1.55-1.63 (2H, m), 3.63 (1H, dt, J=5.6, 5.6), 3.93 (1H, quin., J=6.3), 3.98 (1H, m), 4.01 (2H, t, J=6.9), 5.04 (1H, d, J=5.9), 5.42 (1H, d, J=4.6), 5.73 (1H, d, J=3.0), 7.07 (1H, d, J=7.6), 7.97 (1H, d, J=7.6), 10.66 (1H, br. s)

### Example 36: 5'-deoxy-N⁴-(n-pentyloxycarbonyl)-5-vinylcytidine

MS:LC-MS : (m/z) 368 [M+H]+
¹H-NMR : (270MHz; DMSO-d6) : δ 0.88 (3H, t, J = 7.1), 1.31 (7H, m), 1.61 (2H, m), 3.74 (1H, m), 3.91 (1H, m), 4.06 (2H, t, J = 6.4), 4.22 (1H, m), 5.08 (1H, d, J = 5.3), 5.20 (1H, d, J = 11.3), 5.40 (1H, d, J = 4.9), 5.69 (1H, d, J = 4.0), 5.88 (1H, d, J = 17.9), 6.57 (1H, dd, J = 11.3, 17.9), 7.78 (1H, s), 11.88 (1H, s)

### Example 37: 5'-deoxy-N⁴-(benzyloxycarbonyl)-5-vinylcytidine

MS:FAB-MS : (m/z) 388 [M+H]+, 410 [M+Na]+,
¹H-NMR : (270 MHz; DMSO-d₆) : δ 1.30 (3H, d, J = 6.3), 3.73 (1H, m), 3.92 (1H, m), 4.23 (1H, m), 5.13 (2H, s), 5.04-5.22 (2H, m), 5.42 (1H, d, J = 5.3), 5.69 (1H, d, J = 4.3), 5.69 (1H, dd, J = 15.8, 2.0), 6.55 (1H, dd, J = 11.2, 15.8), 7.36-7.42 (5H, m), 7.78 (1H, s), 11.87 (1H, s).

### Example 38: N⁴-(ethoxycarbonyl)-5'-deoxy-5-vinylcytidine

MS:FAB-MS : (M/Z) 326 [M+H]+, 348 [M+NA]+
¹H-NMR : (270 MHZ; DMSO-D₆) : Δ 1.23 (3H, T, J = 7.26), 1.32 (3H, D, J = 6.27), 3.70-3.76 (1H, M), 3.89-3.94 (1H, M), 4.11 (2H, Q, J = 7.26), 4.22 (1H, M), 5.09 (1H, D, J = 5.61), 5.18-5.22 (1H, M), 5.42 (1H, D, J = 5.61), 5.69 (1H, D, J = 3.96), 5.85-5.92 (1H, M), 6.57 (1H, DD, J =11.88, 17.82), 7.79 (1H, S), 11.88 (1H, BR.S)

### Example 39: 5'-deoxy-5-iodo-N⁴-[(2-phenylethoxy)carbonyl]cytidine

MS:FAB-MS : (m/z) 502 [M+H]+
¹H-NMR : (270MHz; DMSO-d6) : δ 1.30 (3H, d, J = 6.3), 2.96 (2H, t, J = 7.1), 3.69 (1H, m), 3.88 (1H, m), 4.17 (1H, m), 4.29 (2H, t, J = 7.1), 5.07 (1H, d, J = 5.9), 5.38 (1H, d, J = 5.3), 5.62 (1H, d, J = 4.6), 7.19 - 7.35 (5H, m), 8.01 (1H, s), 11.70 (1H, br.s)

### Example 40: 5'-deoxy-5-iodo-N⁴-(isopropoxycarbonyl)cytidine

MS:MALDI-TOF : (m/z) 462.5 [M+Na]+
¹H-NMR : (270MHz; DMSO-d6) : δ 1.24 (6H, d, J = 6.3), 1.30 (3H, d, J = 6.3), 3.69 (1H, m), 3.88 (1H, m), 4.17 (1H, m), 4.87 (1H, m), 5.07 (1H, d, J = 5.6), 5.38 (1H, d, J = 5.3), 5.62 (1H, d, J = 4.3), 8.02 (1H, s), 11.77 (1H, br.s)

### Example 41: N⁴-(cyclohexyloxycarbonyl)-5'-deoxy-5-iodocytidine

MS:LC-MS : (m/z) 479.9 [M+H]+
¹H-NMR : (270MHz; DMSO-d6) : δ 1.23-1.42 (6H, m), 1.29 (3H, d, J = 6.3), 1.70 (2H, m), 1.89 (2H, m), 3.69 (1H, m), 3.88 (1H, m), 4.16 (1H, m), 4.60 (1H, m), 5.05 (1H, d, J = 5.9), 5.37 (1H, d, J = 5.3), 5.62 (1H, d, J = 4.3), 8.00 (1H, s)

The following compounds can also be obtained in a manner analogous to that of Example 6:
5'-deoxy-N⁴-(n-pentyloxycarbonyl)-5-prop-1-ynylcytidine,
5-but-1-ynyl-5'-deoxy-N⁴-(n-pentyloxycarbonyl)cytidine,
5'-deoxy-5-pent-1-ynyl-N⁴-(n-pentyloxycarbonyl)cytidine,
5'-deoxy-5-hex-1-ynyl-N⁴-(n-pentyloxycarbonyl)cytidine,
5'-deoxy-5-bromo-N⁴-(n-pentyloxycarbonyl)cytidine,
5'-deoxy-5-(1-chlorovinyl)-N⁴-(n-pentyloxycarbonyl)cytidine,
5'-deoxy-N⁴-(n-pentyloxycarbonyl)-5-vinylcytidine,
5'-deoxy-5-ethynyl-N⁴-(isopentyloxycarbonyl)cytidine, and
5'-deoxy-N⁴-[(2-ethylbutyl)oxycarbonyl]-5-ethynylcytidine.

### Example 42:

### Preparation of 2',3'-di-O-acetyl-5'-deoxy-5-iodocytidine

5-Iodocytosine (1.0 g; 4.22 mmol) and a catalytic amount of (NH₄)₂SO₄ were suspended in a solution of toluene (10 ml) and hexamethyldisilazane (20 ml). The suspension was heated at 110 °C for 18 hours to become a clear solution. After concentrating the reaction solution under reduced pressure, acetonitrile (25 ml) and 5-deoxy-1,2,3-tri-O-acetyl-D-ribofuranoside (1.32 g; 5.06 mmol) were added to residue. Then, anhydrous stannic chloride(0.58 ml; 5.06 mmol) in nitromethane (5 ml) was added dropwise to the mixture over 5 minutes. During the addtion, the mixture was kept below 0°C by ice cooling. After stirring the mixture at 0 ∼ 5 °C for 2 hours, 2 g of sodium bicarbonate was added, follwed by dropwise addition of water (0.7 ml). After the addtion, the mixture was stirred vigorously at room temperature for 30 minutes. The reaction mixture was filtered to remove insoluble material, which was washed with CH₂Cl₂. The filtrate and washing were combined, and washed with water and sat.aq. sodium bicarbonate, and then dried over Na₂SO₄ and filtered. The filtrate was evaporated under reduced pressure. The crude product was purified by flash chromatography on SiO₂ (eluent : 5% MeOH / CH₂Cl₂ ) to give 5'-deoxy-2',3'-di-O-acetyl-5-iodocytidine as a colorless solid. (1.22 g, 66% yield)
FAB-MS : (m/z) 438[M+H]⁺, 460[M+Na]⁺
1H-NMR : (270MHz;DMSO-d6) : δ 1.32(3H, d, J = 6.3), 2.04(3H, s), 2.06(3H, s), 4.02(1H, quin., J = 6.3), 5.14(1H, t, J = 6.6), 5.48(1H, dd, J = 6.6, 4.3), 5.69(1H, d, J = 4.0), 6.78(1H, br.s), 8.01 (1H, br.s), 8.11(1H, s)

### Example 43:

### Preparation of 2',3'-bis-O-(tert-butyldimethylsilyl)-5'-deoxy-5-iodo-N⁴-(n-pentyloxycarbonyl)cytidine

a) 5'-deoxy-2',3'-di-O-acetyl-5-iodocytidine (200 mg; 0.46mmol) was dissolved in methanol (5 ml). To this solution 1mol/l sodium hydoxide solution was added dropwise at 0°C. After stirring for 10 minutes, the reaction mixture was adjusted to pH 7 with 1N-hydrochloric acid solution. The reaction mixture was evaporated under reduced pressure.
   A mixture of imidazole(467 mg; 6.9 mmol) in DMF(5 ml) was added to the residue. Then tert-butyldimethylchlorosilane(345 mg; 2.29 mmol) was added to the mixture. The reaction mixture was stirred at 50 °C for 1 hour. The mixture was extracted with dichloromethane, washed with water and then dried over Na₂SO₄ and filtered. The filtrate was evaporated under reduced pressure. The crude product was purified by flash chromatography on SiO₂ (eluent :70% EtOAc / n-hexane to 100% EtOAc) to give 5'-deoxy-2',3'-di-O-tert-butyldimethysilyl-5-iodocytidine as a colorless solid. (176.5 mg, 66 % yield)
   FAB-MS : (m/z) 582[M+H]⁺
   1H-NMR : (270MHz;DMSO-d6) δ 0.00 (3H, s), 0.02 (3H, s), 0.06 (3H, s), 0.08 (3H, s), 0.82 (9H, s), 0.88 (9H, s), 1.30 (3H, d, J = 6.6), 3.78 (1H, dd, J = 4.6, 4.3), 3.93 (1H, m), 4.33 (1H, dd, J = 4.9, 4.6), 5.67 (1H, d, J = 5.0), 6.67(1H, br.s), 7.87(2H, br.s)
b) To a stirred solution of 5'-deoxy-2',3'-bis-O-(tert-butyldimethylsilyl)-5-iodocytidine (116 mg, 0.200 mmol) in CH₂Cl₂ (2 ml) pyridine (84 µl, 1.00 mmol), N,N-dimethylamino-pyridine (6 mg, 0.05 mmol), and n-pentyl chloroformate (95 µl, 0.600 mmol) was added at room temperature under Ar. After stifling for 30 min., the reaction mixture was partitioned with dichloromethane and water and the organic phase was separated and the water phase was extracted with CH₂Cl₂ (15 ml x 4). The combined organic phase was washed with water and brine, dried over Na₂SO₄ and filtered. The filtrate was evaporated under reduced pressure. The crude product was purified by flash chromatography on SiO₂ (eluent: 20% EtOAc/n-hexane) to give 2',3'-bis-O-(tert-butyldimethylsilyl)-5'-deoxy-5-iodo-N⁴-(n-pentyloxycarbonyl)cytidine as a colorless amorphous solid. (132.4 mg, 91% yield)
   FAB-MS : (m/z) 696 [M+H]⁺
   ¹H-NMR : (270MHz; DMSO-d₆) : δ 0.00 (3H, s), 0.03 (3H, s), 0.05 (3H, s), 0.07 (3H, s), 0.77 (9H, s), 0.81 (9H, s), 1.20-1.27 (10H, m), 1.46-1.55 (2H, m), 3.74 (1H, dd, J = 4.6, 4.6), 3.89-4.01 (3H, m), 4.37 (1H, dd, J = 4.5, 4.6), 5.55 (1H, d, J = 4.6), 7.92 (1H, s), 11.70 (1H, br.s)

### Example 44:

### Preparation of 2',3'-bis-O-(tert-butyldimethylsilyl)-5'-deoxy-5-[(trimethylsilyl)ethynyl]-N⁴-(n-pentyloxycarbonyl)cytidine

To a solution of 2',3'-bis-O-(tert-butyldimethylsilyl)-5'-deoxy-5-iodo-N⁴-(n-pentyloxycarbonyl)cytidine (130mg, 0.18mmol) in CH₂Cl₂(2ml) and Et₃N(2ml) Cul (10.7mg, 0.1056mmol), Pd(PPh₃)₂Cl₂ (2.6mg, 0.0036mmol), and trimethylsilylacetylene (58.6µl, 0.40mmol) were added and stirred for 2 h at room temperature under Ar in the dark. The reaction mixture was concentrated under reduced pressure and the residue was dissolved in EtOAc(25 mlx3), washed with 2% aq. EDTAo2Na(10mlx2), water and brine, dried over Na₂SO₄ and filtered. The filtrate was evaporated under reduced pressure. The crude product was purified by flash chromatography on SiO₂ (eluent: 10%EtOAc/n-hexane) to give 2',3'-bis-O-(tert-butyldimethylsilyl)-5'-deoxy-5-[(trimethylsilyl)ethynyl]-N⁴-(n-pentyloxycarbonyl)cytidine as a colorless amorphous solid. (30.2 mg, 26% yield)
FAB-MS : (m/z) 666[M+H]⁺, 688[M+Na]⁺
¹H-NMR : (270MHz;DMSO-d6) : δ -0.18 (3H, s), -0.16 (3H, s), -0.14 (3H,s), -0.12 (3H,s), 0.00 (9H,s), 0.64 (9H, s), 0.65 (3H, s), 0.67 (9H, s), 1.01(4H, m), 1.14 (3H, d, J = 6.6), 1.40 (2H, m), 3.58 (1 H, t, J = 4.9), 3.79 (1 H, m), 3.87 (2H, m), 4.20 (1 H, m), 5.43 (1 H, d, J = 3.6), 7.88 (1 H, br.s)

### Example 45:

### 5'-deoxy-2',3'-bis-O-(tert-butyldimethylsilyl)-5-cyanocytidine

To a stirred solution of 5'- deoxy-2', 3'-bis-(O-tert-butyldimethylsilyloxy)-5-iodocytidine (153 mg, 0.263 mmol) in DMF (5 ml) NaCN (34.3 mg, 0.70 mmol) was added at room temperature. After stirring for 1 day, the reaction mixture was concentrated under reduced pressure. The crude product was dissolved in EtOAc, and then washed with water and brine. The extract was dried over Na₂SO₄ and filtered. The filtrate was concentrated under reduced pressure. The crude product was purified by flash chromatography on SiO₂ (eluent : EtOAc) to give 5'-deoxy-2',3'-bis-O-(tert-butyldimethylsilyl)-5-cyanocytidine as a pale yellow solid. (71.1 mg, 56 % yield)
FAB-MS : (m/z) 481[M+H]⁺
¹H-NMR : (270MHz; DMSO-d₆) : δ -0.04 (3H, s), 0.00 (3H, s), 0.02 (3H,s), 0.76 (9H, s), 0.82 (9H, s), 1.21 (3H, d, J = 6.3), 3.81 (1 H, m), 4.05 (1H, t, J = 5.0), 4.71 (1 H, t , J = 5.0), 5.65 (1 H, d, J = 5.3), 6.41 (1 H, s), 7.69 (1 H, br.s), 7.85 (1 H, br.s)

### Reference-Example 46 : Preparation of 2',3'-di-O-acetyl-5'-deoxy-5-vinylcytidine

To a solution of 2',3'-di-O-acetyl-5'-deoxy-5-iodocytidine (1.6 g, 3.66 mmol) in 10 ml DMF were added Pd2(dba)₃ (67 mg, 0.073 mmol) and tri-2-furylphosphine (85mg, 0.366mmol) and tri-n-butyl(vinyl)stannane (2.1ml, 7.318mmol ) under Ar atmosphere at room temperature. After stirring for 19h, tri-n-butyl(vinyl)stannane ( 2.1ml, 7.318mmol) was added to the reaction mixture, and then the reaction mixture was warmed up to 40∞C with stirring for 24hr. The solvent was removed in vacuo, and the residue was purified on a column of silica gel ( eluent : ethyl acetate ∼ CH₂Cl₂ : MeOH = 95 : 5) to give 2',3'-di-O-acetyl-5'-deoxy-5-vinylcytidine (1.13g, 92 %) as colorless solid:
MS:FABMS : (m/z) 338 [M+H]+
¹H-NMR : ( 270MHz; DMSO-d₆) : d 1.33 ( 3H, d, *J* = 6.3 ), 2.05 ( 3H, s ), 2.06 ( 3H, s ), 4.05 ( 1H, quin., *J* = 6.3), 5.14 ( 1H, d, *J* = 10.8 ), 5.16 ( 1H, t, *J* = 6.6 ), 5.54 ( 1H, d, *J* = 17.2 ), 5.53 (1H, dd, *J* = 6.9, 5.9 ), 5.73 ( 1H, d, *J* = 4.3 ), 6.55 ( 1H, dd, *J* = 17.2, 10.8 ), 7.20 ( 1H, br. s ). 7.57 ( 1H, br. s ), 7.88 ( 1H, s )

### Example 47: Preparation of 5'-deoxy-5-vinylcytidine

To a solution of 2',3'-di-O-acetyl-5'-deoxy-5-vinylcytidine (111 mg, 3.29 mmol) in 5 ml of methanol was added 1N NaOH (0.32 ml, 0.32 mmol) at room temperature. After stirring for 1 h, 1N HCl (ca.0.3 ml) was added to the reaction mixture, and then the reaction mixture was concentrated under reduced pressure. The residue was purified by solid phase extraction ( MEGA Bond Elute LRC, eluent : H₂O ∼ H₂O : MeOH = 1 : 1, step gradient) to give 5'-deoxy-5-vinylcytidine (82 mg, 98 %) as colorless solid:
MS:LC-MS : (m/z) 253.9 [M+H]+
¹H-NMR : ( 270MHz; DMSO-d6 ) : d 1.29 ( 3H, d, J = 6.3 ), 3.68 ( 1H, m ), 3.86 ( 1H, m ), 4.08 ( 1H, m ), 4.97 ( 1H, d, J = 5.9 ), 5.12 ( 1H, d, J = 11.1 ), 5.28 ( 1H, d, J = 5.3 ), 5.50 ( 1H, d, J = 17.2 ), 5.70 ( 1H, d, J = 3.6 ), 6.58 ( 1H, dd, J = 11.1, 17.2 ), 7.10 ( 1H, br.s ), 7.42 ( 1H, br.s ), 7.64 ( 1H, s )

The following examples illustrate pharmaceutical preparations containing a compound provided by the present invention.

### Example A:

Interlocking gelatin capsules each containing the following ingredients were manufactured in a manner known *per se*:

| | |
|---|---|
| 5'-Deoxy-5-ethynyl-N⁴-(n-pentyloxycarbonyl)-cytidine | 40 mg |
| Lactose | 70 mg |
| Corn starch | 25 mg |
| Magnesium stearate | 1 mg |
| Crospovidone | 4 mg |
| | 140 mg |

### Example B:

Interlocking gelatin capsules each containing the following ingredients were manufactured in a manner known *per se*:

| | |
|---|---|
| 5'-Deoxy-5-fluoro-N⁴-(n-pentyloxycarbonyl)-cytidine | 100 mg |
| 5'-Deoxy-5-ethynyl-N⁴-(n-pentyloxycarbonyl)-cytidine | 10 mg |
| Lactose | 70 mg |
| Corn starch | 25 mg |
| Magnesium stearate | 1 mg |
| Crospovidone | 4 mg |
| | 210 mg |

### Example C:

Tablets each containing the following ingredients were manufactured in a manner known *per se*:

| | |
|---|---|
| 5'-Deoxy-5-ethynyl-N⁴-(n-pentyloxycarbonyl)-cytidine | 40 mg |
| Lactose | 70 mg |
| Magnesium stearate | 3 mg |
| Crospovidone | 7 mg |
| Povidone | 10 mg |
| | 130 mg |

If necessary, the tablet is film-coated with hydroxypropylmethyl cellulose, talc and colorant.

### Example D:

Tablets each containing the following ingredients were manufactured in a manner known *per se*:

| | |
|---|---|
| 5'-Deoxy-5-fluoro-N⁴-(n-pentyloxycarbonyl) -cytidine | 300 mg |
| 5'-Deoxy-5-ethynyl-N⁴-(n-pentyloxycarbonyl)-cytidine | 20 mg |
| Lactose | 70 mg |
| Magnesium stearate | 3 mg |
| Crospovidone | 7 mg |
| Povidone | 10 mg |
| | 186 mg |

If necessary, the tablet is film-coated with hydroxypropylmethyl cellulose, talc and colorant.

## Claims

1. A compound represented by the general formula (I) 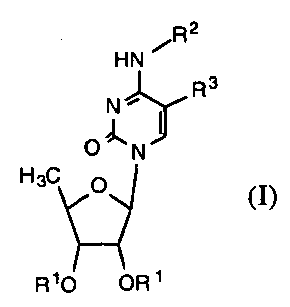 wherein R¹ is a hydrogen atom or a group selected from acetyl, propionyl, benzoyl, toluoyl, glycyl, alanyl, β-alanyl, valyl, lysyl; R² is a hydrogen atom, or-CO-OR⁴ group [wherein R⁴ is a saturated or unsaturated, straight or branched hydrocarbon group consisting of one to fifteen carbon atoms, or a group of the formula -(CH₂)ₙ-Y (in which Y is cyclohexyl or phenyl; n is an integer from 0 to 4)]; R³ is a hydrogen atom, bromo, iodo, trifluoromethyl, ethyl, propyl, cyano, vinyl, 1-chlorovinyl, ethynyl, prop-1-ynyl, but-1-ynyl, pent-1-ynyl, hex-1-ynyl, or bromoethynyl; with the proviso that R² and R³ do not mean a hydrogen atom at the same time, and formula (I) does not represent 2',3'-di-O-acetyl-5'-deoxy-5-vinylcytidine.

2. A compound of claim 1 selected from the group consisting of:
5'-deoxy-5-ethynylcytidine,
5'-deoxy-5-prop-1-ynylcytidine,
5-but-1-ynyl-5'-deoxycytidine,
5'-deoxy-5-pent-1-ynylcytidine,
5'-deoxy-5-hex-1-ynylcytidine,
5'-deoxy-5-iodocytidine,
5-bromo-5'-deoxycytidine,
5-(1-chlorovinyl)-5'-deoxycytidine,
5'-deoxy-5-vinylcytidine,
5'-deoxy-5-trifluoromethylcytidine
5-cyano-5'-deoxycytidine,
5'-deoxy-N⁴-(n-pentyloxycarbonyl)cytidine,
5'-deoxy-N⁴-(n-pentyloxycarbonyl)-5-prop-1-ynylcytidine,
5-but-1-ynyl-5'-deoxy-N⁴-(n-pentyloxycarbonyl)cytidine,
5'-deoxy-5-pent-1-ynyl-N⁴-(n-pentyloxycarbonyl)cytidine,
5'-deoxy-5-hex-1-ynyl-N⁴-(n-pentyloxycarbonyl)cytidine,
5'-deoxy-5-iodo-N⁴-(n-pentyloxycarbonyl)cytidine,
5-bromo-5'-deoxy-N⁴-(n-pentyloxycarbonyl)cytidine,
5-(1-chlorovinyl)-5'-deoxy-N⁴-(n-pentyloxycarbonyl)cytidine,
N⁴-(ethoxycarbonyl)-5'-deoxy-5-vinylcytidine,
5'-deoxy-N⁴-(n-propoxycarbonyl)-5-vinylcytidine,
N⁴-(n-butoxycarbonyl)-5'-deoxy-5-vinylcytidine,
5'-deoxy-N⁴-(n-pentyloxycarbonyl)-5-vinylcytidine,
N⁴-(benzyloxycarbonyl)-5'-deoxy-5-vinylcytidine,
5'-deoxy-N⁴-(n-pentyloxycarbonyl)-5-trifluoromethylcytidine,
5-cyano-5'-deoxy-N⁴-(n-pentyloxycarbonyl)cytidine,
5'-deoxy-5-ethynyl-N⁴-(methoxycarbonyl)cytidine
5'-deoxy-N⁴-(ethoxycarbonyl)-5-ethynylcytidine
5'-deoxy-5-ethynyl-N⁴-(n-propoxycarbonyl)cytidine,
5'-deoxy-5-ethynyl-N⁴-(isopropoxycarbonyl)cytidine,
N⁴-(n-butoxycarbonyl)-5'-deoxy-5-ethynylcytidine,
5'-deoxy-5-ethynyl-N⁴-(isobutoxycarbonyl)cytidine,
5'-deoxy-5-ethynyl-N⁴-(n-pentyloxycarbonyl)cytidine,
5'-deoxy-5-ethynyl-N⁴-[(2-propylpentyloxy)carbonyl]cytidine,
5'-deoxy-5-ethynyl-N⁴-(isopentyloxycarbonyl)cytidine,
5'-deoxy-5-ethynyl-N⁴-[(2-methylpentyloxy)carbonyl]cytidine,
5'-deoxy-5-ethynyl-N⁴-[(3-methylpentyloxy)carbonyl]cytidine,
5'-deoxy-5-ethynyl-N⁴-(n-hexyloxycarbonyl)cytidine,
5'-deoxy-N⁴-[(2-ethylbutyl)oxycarbonyl]-5-ethynylcytidine,
5'-deoxy-N⁴-[(2-ethylhexyl)oxycarbonyl]-5-ethynylcytidine,
5'-deoxy-5-ethynyl-N⁴-[(2-phenylethoxy)carbonyl]cytidine,
N⁴-(cyclohexyloxycarbonyl)-5'-deoxy-5-ethynylcytidine,
N⁴-[(cyclohexylmethoxy)carbonyl]-5'-deoxy-5-ethynylcytidine,
5'-deoxy-5-ethynyl-N⁴-(neopentyloxycarbonyl)cytidine,
5'-deoxy-N⁴-[(3,3-dimethylbutoxy)carbonyl]-5-ethynylcytidine,
2',3'-di-O-acetyl-5'-deoxy-5-ethynyl-N⁴-(n-propoxycarbonyl)cytidine and
2',3'-di-O-acetyl-5'-deoxy-5-ethynyl-N⁴-(n-pentyloxycarbonyl)-cytidine
2',3'-di-O-acetyl-N⁴-(ethoxycarbonyl)-5'-deoxy-5-vinylcytidine
2',3'-di-O-acetyl-5'-deoxy-N⁴-(n-propoxycarbonyl)-5-vinylcytidine,
2',3'-di-O-acetyl-N⁴-(n-butoxycarbonyl)-5'-deoxy-5-vinylcytidine,
2',3'-di-O-acetyl-5'-deoxy-N⁴-(n-pentyloxycarbonyl)-5-vinylcytidine, and
2',3'-di-O-acetyl-N⁴-(benzyloxycarbonyl)-5'-deoxy-5-vinylcytidine.
5'-deoxy-5-ethynyl-N⁴-(n-decyloxycarbonyl)cytidine
5'-deoxy-5-ethynyl-N⁴-[(2,6-dimethylcyclohexyloxy)-carbonyl]cytidine
5'-deoxy-5-ethynyl-N⁴-(benzyloxycarbonyl)cytidine
5'-deoxy-5-ethynyl-N⁴-[(1-isopropyl-2-methyl-propoxy)carbonyl]cytidine
5'-deoxy-5-ethynyl-N⁴-[(3-methoxybenzyloxy)-carbonyl]cytidine.

3. A compound as defined in claim 1 or 2 for use in medical therapy.

4. A compound as defined in claim 1 or 2 for use in treatment of tumor.

5. A pharmaceutical composition comprising a compound as defined in claim 1 or 2 as an active ingredient.

6. A pharmaceutical composition for the treatment of tumor comprising a compound as defined in claim 1 or 2 and an active ingredient.

7. A pharmaceutical composition comprising a compound of claim 1 or 2 and 5-fluorouracil or a derivative thereof.

8. A pharmaceutical composition comprising 2',3'-di-O-acetyl-5'-deoxy-5-vinylcytidine and 5-fluorouracil or a derivative thereof.

9. A composition of claim 7 or 8, wherein the 5-fluorouracil or its derivative is selected from the group consisting of:
5-fluoro-1-(2-tetrahydrofuryl)uracil,
1-(n-hexyloxycarbonyl)-5-fluorouracil,
5'-deoxy-5-fluorouridine,
5'-deoxy-5-fluoro-N⁴-(n-propoxycarbonyl)cytidine,
N⁴-(n-butoxycarbonyl)-5'-deoxy-5-fluorocytidine,
5'-deoxy-5-fluoro-N⁴-(n-pentyloxycarbonyl)cytidine,
5'-deoxy-5-fluoro-N⁴-(isopentyloxycarbonyl)cytidine,
5'-deoxy-5-fluoro-N⁴-(n-hexyloxycarbonyl)cytidine,
5'-deoxy-N⁴-[(2-ethylbutyl)oxycarbonyl]-5-fluorocytidine,
5'-deoxy-5-fluoro-N⁴-[(2-phenylethoxy)carbonyl]cytidine,
N⁴-[(cyclohexylmethoxy)carbonyl]-5'-deoxy-5-fluorocytidine,
5'-deoxy-5-fluoro-N4-(neopentyloxycarbonyl)-cytidine,
5'-deoxy-N⁴-[(3,3-dimethylbutoxy)carbonyl]-5-fluorocytidine,
5'-deoxy-5-fluoro-N⁴-(3,5-dimethylbenzoyl)cytidine,
5'-deoxy-5-fluoro-N⁴-(3,5-dichlorobenzoyl)cytidine, and 2',3'-di-O-acetyl-5'-deoxy-5-fluoro-N⁴-(n-pentyloxycarbonyl)cytidine.

10. A pharmaceutical composition of claim 7 or 8 for treatment of tumor.

11. Use of a compound as defined in claim 1 or 2 in the manufacture of medicament for the treatment of tumor.

12. A kit comprising a pharmaceutical composition containing a compound as defined in claim 1 or 2 as an active ingredient and a pharmaceutical composition containing 5-fluorouracil or a derivative thereof as an active ingredient.

13. A process for manufacturing a compound represented by the general formura (I), 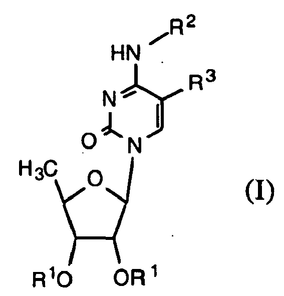 wherein R¹ is a hydrogen atom or a group selected from acetyl, propionyl, benzoyl, toluoyl, glycyl, alanyl, β-alanyl, valyl, lysyl; R² is a hydrogen atom, or -CO-OR⁴ group [wherein R⁴ is a saturated or unsaturated, straight or branched hydrocarbon group consisting of one to fifteen carbon atoms, or a group of the formula -(CH₂)ₙ-Y (in which Y is cyclohexyl or phenyl; n is an integer from 0 to 4)]; R³ is a hydrogen atom, bromo, iodo, trifluoromethyl, ethyl, propyl, cyano, vinyl, 1-chlorovinyl, ethynyl, prop-1-ynyl, but-1-ynyl, pent-1-ynyl, hex-1-ynyl, or bromoethynyl; with the proviso that R² and R³ do not mean a hydrogen atom at the same time, and formula (I) does not represent 2',3'-di-O-acetyl-5'-deoxy-5-vinylcytidine
which comprises:
(A) for a compound of the general formula (I) wherein R¹, R² and R³ are the same as defined above, reacting a compound represented by the formula (II), 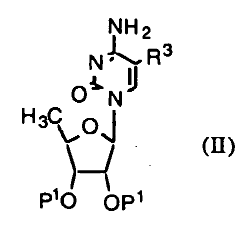 wherein P¹ is a hydroxy-protecting group and R³ is the same as defined above,
with a compound represented by the general formula (III),
R⁴OCOX (III)
wherein R⁴ is the same as defined above; X is chloro or bromo, in the presence of acid acceptor, followed, if necessary, by removal of protecting group(s),
(B) for a compound represented by the formula (I), wherein R¹ and R² are the same as defined above and R³ is vinyl, 1-chlorovinyl, ethynyl, prop-1-ynyl, but-1-ynyl, pent-1-ynyl, hex-1-ynyl, or bromoethynyl, reacting a compound represented by the formula (IV) 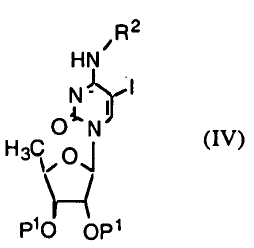 wherin P¹ and R² are the same as defined above, with an acetylene or vinyl derivative in the presence of a palladium catalyst, followed, if necessary, by removal of protecting group(s),
(C) for a compound represented by the formula (I) wherein R¹ and R² are the same as defined above, and R³ is a cyano group, reacting a compound represented by the formula (IV) 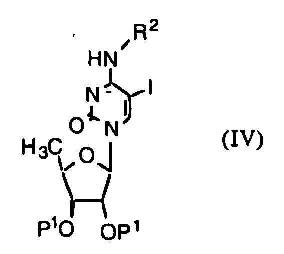 wherein P¹ and R² is the same as defined above, with alkali metal cyanide, followed, if necessary, by removal of protecting group(s),
(D) for a compound represented by the formula (I), wherein R¹ and R³ are the same as defined above and R² is a hydrogen atom, reacting a compound represented by the formula (V) 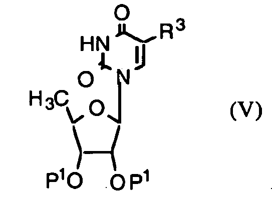 wherein P¹ and R³ is the same as defined above, with phosphoryl chloride in the presence of an acid acceptor, followed by treatment with ammonia,
followed, if necessary, by removal of protecting group(s),
(E) for a compound represented by the formula (I), wherein R¹, R² and R³ are the same as defined above, coupling a compound represented by the formula (VI) 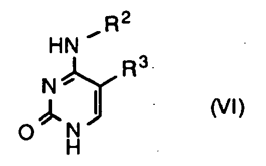 wherein R² and R³ are the same as defined above, with a compound represented by the formula (VII) 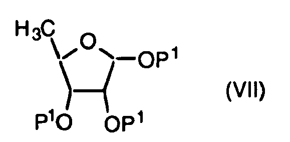 wherein P1 is the same as defined above, in the presence of Lewis acid catalyst, followed, if necessary, by removal of protecting group(s).
(F) for a compound represented by the formula (I) wherein R3 is vinyl, 1-chlorovinyl, R¹ and R² are the same as defined above, catalytic hydrogenation of a compound represented by the formula (VIII) 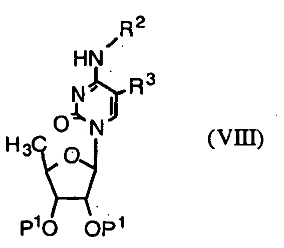 [wherein P¹ is a hydroxy-protecting radical, R³ is an ethynyl or 1-chloroethynyl radical, and R² is the same as defined above],
with Lindlar catalyst, followed, if necessary, by removal of protecting radical(s).

## Patentansprüche

1. Verbindung der allgemeinen Formel (I) 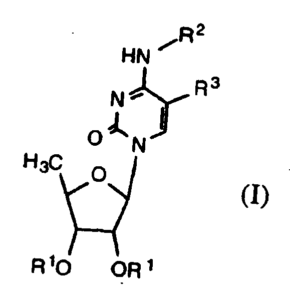 worin R¹ ein Wasserstoffatom oder eine Gruppe, ausgewählt aus Acetyl, Propionyl, Benzoyl, Toluoyl, Glycyl, Alanyl, β-Alanyl, Valyl, Lysyl ist; R² ein Wasserstoffatom oder eine -CO-OR⁴-Gruppe ist [worin R⁴ eine gesättigte oder ungesättigte, gerade oder verzweigte Kohlenwasserstoffgruppe bestehend aus 1 bis 15 Kohlenstoffatomen oder eine Gruppe der Formel -(CH₂)ₙ-Y ist (worin Y Cyclohexyl oder Phenyl ist; n eine ganze Zahl von 0 bis 4 ist)]; R³ ein Wasserstoffatom, Brom, Iod, Trifluormethyl, Ethyl, Propyl, Cyano, Vinyl, 1-Chlorvinyl, Ethinyl, Prop-1-inyl, But-1-inyl, Pent-1-inyl, Hex-1-inyl oder Bromethinyl ist; mit dem Vorbehalt, dass R² und R³ nicht gleichzeitig ein Wasserstoffatom bedeuten, und Formel (I) nicht 2',3'-Di-O-acetyl-5'-desoxy-5-vinylcytidin darstellt.

2. Verbindung nach Anspruch 1 ausgewählt aus der Gruppe bestehend aus
5'-Desoxy-5-ethinylcytidin,
5'-Desoxy-5-prop-1-inylcytidin,
5-But-1-inyl-5'-desoxycytidin,
5'-Desoxy-5-pent-1-inylcytidin,
5'-Desoxy-5-hex-1-inylcytidin,
5'-Desoxy-5-iodcytidin,
5-Brom-5'-desoxycytidin,
5-(1-Chlorvinyl)-5'-desoxycytidin,
5'-Desoxy-5-vinylcytidin,
5'-Desoxy-5-trifluormethylcytidin,
5-Cyano-5'-desoxycytidin,
5'-Desoxy-N⁴-(n-pentyloxycarbonyl)cytidin,
5'-Desoxy-N⁴-(n-pentyloxycarbonyl)-5-prop-1-inylcytidin,
5-But-1-inyl-5'-desoxy-N⁴-(n-pentyloxycarbonyl)cytidin,
5'-Desoxy-5-pent-1-inyl-N⁴-(n-pentyloxycarbonyl)cytidin,
5'-Desoxy-5-hex-1-inyl-N⁴-(n-pentyloxycarbonyl)cytidin,
5'-Desoxy-5-iod-N'-(n-pentyloxycarbonyl)cytidin,
5-Brom-5'-desoxy-N⁴-(n-pentyloxycarbonyl)cytidin,
5-(1-Chlorvinyl)-5'-desoxy-N⁴-(n-pentyloxycarbonyl)cytidin,
N⁴-(Ethoxycarbonyl)-5'-desoxy-5-vinylcytidin,
5'-Desoxy-N⁴-(n-propoxycarbonyl)-5-vinylcytidin,
N⁴-(n-Butoxycarbonyl)-5'-desoxy-5-vinylcytidin,
5'-Desoxy-N⁴-(n-pentyloxycarbonyl)-5-vinylcytidin,
N⁴-(Benzyloxycarbonyl)-5'-desoxy-5-vinylcytidin,
5'-Desoxy-N⁴-(n-pentyloxycarbonyl)-5-trifluormethylcytidin,
5-Cyano-5'-desoxy-N⁴-(n-pentyoxycarbonyl)cytidin,
5'-Desoxy-5-ethinyl-N⁴-(methoxycarbonyl)cytidin,
5'-Desoxy-N⁴-(ethoxycarbonyl)-5-ethinylcytidin,
5'Desoxy-5-ethinyl-N⁴-(n-propoxycarbonyl)cytidin,
5'-Desoxy-5-ethinyl-N⁴-(isopropoxycarbonyt)cytidin,
N⁴-(n-Butoxycarbonyl)-5'-desoxy-5-ethinylcytidin,
5'-Desoxy-5-ethinyl-N⁴-(isobutoxycarbonyl)cytidin,
5'-Desoxy-5-ethinyl-N⁴-(n-pentyloxycarbonyl)cytidin,
5'-Desoxy-5-ethinyl-N⁴-[(2-propylpentyloxy)carbonyl]cytidin,
5'-Desoxy-5-ethinyl-N⁴-(isopentyloxycarbonyl)cytidin,
5'-Desoxy-5-ethinyl-N⁴-[(2-methylpentlyoxy)carbonyl]cytidin,
5'-Desoxy-5-ethinyl-N⁴-[(3-methylpentyloxy)carbonyl]cytidin,
5'-Desoxy-5-ethinyl-N⁴-(n-hexyloxycarbonyl)cytidin,
5'-Desoxy-N⁴-[(2-ethylbutyl)oxycarbonyl]-5-ethinylcytidin,
5'-Desoxy-N⁴-[(2-ethylhexyl)oxycarbonyl]-5-ethinylcytidin,
5'-Desoxy-5-ethinyl-N⁴-[(2-phenylethoxy)carbonyl]cytidin,
N⁴-(Cyclohexyloxycarbonyl)-5'-desoxy-5-ethinylcytidin,
N⁴-[(Cyclohexylmethoxy)carbonyl]-5'-desoxy-5-ethinylcytidin,
5'-Desoxy-5-ethinyl-N⁴-(neopentyloxycarbonyl)cytidin,
5'-Desoxy-N⁴-[(3,3-dimethylbutoxy)carbonyl]-5-ethinylcytidin,
2',3'-Di-O-acetyl-5'-desoxy-5-ethinyl-N⁴-(n-propoxycarbonyl)cytidin und
2',3'-Di-O-acetyl-5'-desoxy-5-ethinyl-N⁴-(n-pentyloxycarbonyl)-cytidin,
2',3'-Di-O-acetyl-N⁴-(ethoxycarbonyl)-5'-desoxy-5-vinylcytidin,
2'.3'-Di-O-acetyl-5'-desoxy-N⁴-(n-propoxycarbonyl)-5-vinylcytidin,
2',3'-Di-O-acetyl-N⁴-(n-butoxycarbonyl)-5'-desoxy-5-vinylcytidin,
2',3'-Di-O-acetyl-5'-desoxy-N⁴-(n-pentyloxycarbonyl)-5-vinylcytidin, und
2',3'-Di-O-acetyl-N⁴-(benzyloxycarbonyl)-5'-desoxy-5-vinylcytidin.
5'-Desoxy-5-ethinyl-N⁴-(n-decyloxycarbonyl)cytidin
5'-Desoxy-5-ethinyl-N⁴-[(2,6-dimethylcyclohexyloxy)-carbonyl]cytidin
5'-Desoxy-5-ethinyl-N⁴-(benzyloxycarbonyl)cytidin
5'-Desoxy-5-ethinyl-N⁴-[(1-isopropyl-2-methyl-propoxy)carbonyl]cytidin
5'-Desoxy-5-ethinyl-N⁴-[(3-methoxybenzyloxy)-carbonyl]cytidin.

3. Verbindung nach einem der Ansprüche 1 oder 2 zur Verwendung in der medizinischen Therapie.

4. Verbindung nach einem der Ansprüche 1 oder 2 zur Verwendung zur Behandlung eines Tumors.

5. Pharmazeutische Zusammensetzung enthaltend eine Verbindung, wie in einem der Ansprüche 1 oder 2 definiert, als aktiven Inhaltsstoff.

6. Pharmazeutische Zusammensetzung zur Behandlung eines Tumors enthaltend eine Verbindung, wie in Anspruch 1 oder 2 definiert, und einen aktiven Inhaltsstoff.

7. Pharmazeutische Zusammensetzung enthaltend eine Verbindung nach einem der Ansprüche 1 oder 2 und 5-Fluoruracil oder ein Derivat davon.

8. Pharmazeutische Zusammensetzung enthaltend 2',3'-Di-O-acetyl-5'-desoxy-5-vinylcytidin und 5-Fluoruracil oder ein Derivat davon.

9. Zusammensetzung nach Anspruch 7 oder 8, worin das 5-Fluoruracil oder sein Derivat ausgewählt ist aus der Gruppe bestehend aus:
5-Fluor-1-(2-tetrahydrofuryl)uracil,
1-(n-Hexyloxycarbonyl)-5-fluoruracil,
5'-Desoxy-5-fluoruridin,
5'-Desoxy-5-fluor-N⁴-(n-propoxycarbonyl)cytidin,
N⁴-(n-butoxycarbonyl)-5'-desoxy-5-fluorcytidin,
5'-Desoxy-5-fluor-N⁴-(n-pentyloxycarbonyl)cytidin,
5'-Desoxy-5-fluor-N⁴-(isopentyloxycarbonyl)cytidin,
5'-Desoxy-5-fluor-N⁴-(n-hexyloxycarbonyl)cytidin,
5'-Desoxy-N⁴-[(2-ethylbutyl)oxycarbonyl]-5-fluorcytidin,
5'-Desoxy-5-fluor-N⁴-[(2-phenylethoxy)carbonyl]cytidin,
N⁴-[(Cyclohexylmethoxy)carbonyl]-5'-desoxy-5-fluorcytidin,
5'-Desoxy-5-fluor-N4-(neopentyloxycarbonyl)-cytidin,
5'-Desoxy-N⁴-[(3,3-dimethylbutoxy)carbonyl)-5-fluorcytidin,
5'-Desoxy-5-fluor-N⁴-(3,5-dimethylbenzoyl)cytidin,
5'-Desoxy-5-fluor-N⁴-(3,5-dichlorbenzoyl)cytidin, und
2',3'-Di-O-acetyl-5'-desoxy-5-fluor-N⁴-(n-pentyloxycarbonyl)cytidin.

10. Pharmazeutische Zusammensetzung nach Anspruch 7 oder 8 zur Behandlung eines Tumors.

11. Verwendung einer Verbindung nach einem der Ansprüche 1 oder 2 zur Herstellung eines Arzneimittels zur Behandlung eines Tumors.

12. Kit umfassend eine pharmazeutische Zusammensetzung enthaltend eine Verbindung nach einem der Ansprüche 1 oder 2 als Wirkstoff und eine pharmazeutische Zusammensetzung enthaltend 5-Fluoruracil oder ein Derivat davon als Wirkstoff.

13. Verfahren zur Herstellung einer Verbindung der allgemeinen Formel (I), 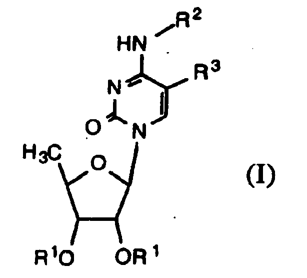 worin R¹ ein Wasserstoffatom oder eine Gruppe ausgewählt aus Acetyl, Propionyl, Benzoyl, Toluoyl, Glycyl, Alanyl, β-Alanyl, Valyl, Lysyl ist; R² ein Wasserstoffatom oder eine -CO-OR⁴-Gruppe ist [worin R⁴ eine gesättigte oder ungesättigte, gerade oder verzweigte Kohlenwasserstoffgruppe bestehend aus 1 bis 15 Kohlenstoffatomen oder eine Gruppe der Formel -(CH₂)ₙ-Y ist (worin Y Cyclohexyl oder Phenyl ist; n eine ganze Zahl von 0 bis 4 ist)]; R³ ein Wasserstoffatom, Brom, Iod, Trifluormethyl, Ethyl, Propyl, Cyano, Vinyl, 1-Chlorvinyl, Ethinyl, Prop-1-inyl, But-1-inyl, Pent-1-inyl, Hex-1-inyl oder Bromethinyl ist; mit dem Vorbehalt dass R² und R³ nicht gleichzeitig ein Wasserstoffatom bedeuten, und Formel (I) nicht 2',3'-Di-O-acetyl-5'-desoxy-5-vinylcytidin darstellt, das umfasst, dass man
(A) für eine Verbindung der allgemeinen Formel (I), worin R¹, R² und R³ wie oben definiert sind, eine Verbindung der Formel (II) 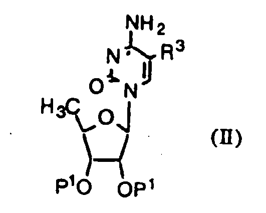 worin P¹ eine Hydroxyschutzgruppe ist und R³ wie oben definiert ist,
mit einer Verbindung der allgemeinen Formel (III)
R⁴OCOX (III)
worin R⁴ wie oben definiert ist, X Chlor oder Brom ist, in Gegenwart eines Säureakzeptors umsetzt und anschließend, falls notwendig, die Schutzgruppe(n) entfernt,
(B) für eine Verbindung der Formel (I), worin R¹ und R² wie oben definiert sind und R³ Vinyl, 1-Chlorvinyl, Ethinyl, Prop-1-inyl, But-1-inyl, Pent-1-inyl, Hex-1-inyl oder Bromethinyl ist, eine Verbindung der Formel (IV) 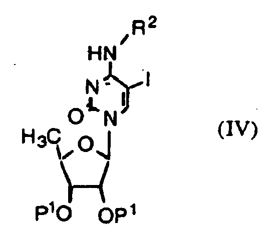 worin P¹ und R² wie oben definiert sind, mit einem Acetylen- oder Vinylderivat in Gegenwart eines Palladiumkatalysators umsetzt und anschließend, falls notwendig, die Schutzgruppe(n) entfernt,
(C) für eine Verbindung der Formel (I), worin R₁ und R² wie oben definiert sind und R³ eine Cyanogruppe ist, eine Verbindung der Formel (IV) 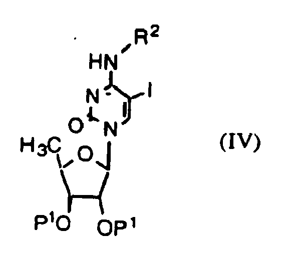 worin P¹ und R² wie oben definiert sind, mit Alkalicyanid umsetzt und anschließend, falls notwendig, die Schutzgruppe(n) entfernt,
(D) für eine Verbindung der Formel (I), worin R¹ und R³ wie oben definiert sind und R² ein Wasserstoffatom ist, eine Verbindung der Formel (V) 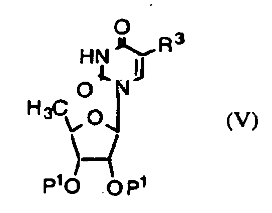 worin P¹ und R³ wie oben definiert sind, mit Phosphorylchlorid in Gegenwart eines Säureakzeptors umsetzt, anschließend mit Ammoniak behandelt und, falls notwendig, anschließend die Schutzgruppe(n) entfernt,
(E) für eine Verbindung der Formel (I), worin R¹, R² und R³ wie oben definiert sind, eine Verbindung der Formel (VI) 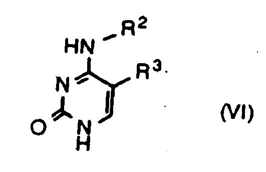 worin R² und R³ wie oben definiert sind, mit einer Verbindung der Formel (VII) 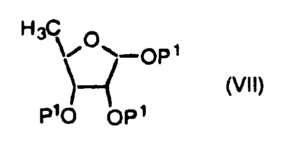 worin P¹ wie oben definiert ist, in Gegenwart eines Lewissäurekatalysators kuppelt und anschließend, falls notwendig, die Schutzgruppe(n) entfernt,
(F) für eine Verbindung der Formel (I), worin R³ Vinyl, 1-Chlorvinyl ist, R¹ und R² wie oben definiert sind, eine Verbindung der Formel (VIII) 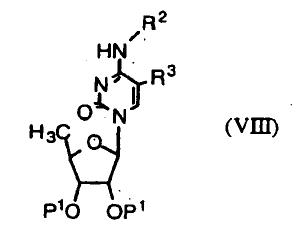 [worin P¹ eine Hydroxyschutzgruppe ist, R³ ein Ethinylrest ist, und R² wie oben definiert ist] mit einem Lindlarkatalysator katalytisch hydriert und, falls notwendig, anschließend die Schutzgruppe(n) entfernt.

## Revendications

1. Composé représenté par la formule générale (I) 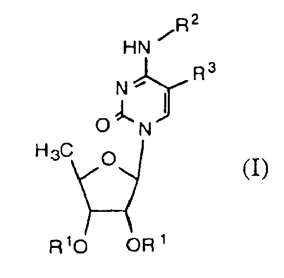 dans laquelle R¹ est un atome d'hydrogène ou un groupe choisi parmi les groupes acétyle, propionyle, benzoyle, toluoyle, glycyle, alanyle, β-alanyle, valyle, lysyle ; R² est un atome d'hydrogène, ou un groupe -CO-OR⁴ [dans lequel R⁴ est un groupe hydrocarboné saturé ou insaturé, à chaîne droite ou ramifiée, comportant de un à quinze atomes de carbone, ou un groupe de formule -(CH₂)ₙ-Y (dans lequel Y est un groupe cyclohexyle ou phényle ; n est un nombre entier de 0 à 4)] ; R³ est un atome d'hydrogène, de brome, d'iode, ou un groupe trifluorométhyle, éthyle, propyle, cyano, vinyle, 1-chlorovinyle, éthynyle, prop-1-ynyle, but-1-ynyle, pent-1-ynyle, hex-1-ynyle ou bromoéthynyle ; à la condition que R² et R³ ne représentent pas simultanément des atomes d'hydrogène, et que la formule (I) ne représente pas la 2',3'-di-O-acétyl-5'-désoxy-5-vinylcytidine.

2. Composé selon la revendication 1 choisi dans groupe constitué par les composés suivants :
5'-désoxy-5-éthynylcytidine,
5'-désoxy-5-prop-1-ynylcytidine,
5-but-1-ynyl-5'-désoxycytidine,
5'-désoxy-5-pent-1-ynylcytidine,
5'-désoxy-5-hex-1-ynylcytidine,
5'-désoxy-5-iodocytidine,
5-bromo-5'-désoxycytidine,
5-(1-chlorovinyl)-5'-désoxycytidine,
5'-désoxy-5-vinylcytidine,
5'-désoxy-5-trifluorométhylcytidine,
5-cyano-5'-désoxycytidine,
5'-désoxy-N⁴-(n-pentyloxycarbonyl)cytidine,
5'-désoxy-N⁴-(n-pentyloxycarbonyl)-5-prop-1-ynylcytidine,
5-but-1-ynyl-5'-désoxy-N⁴-(n-pentyloxycarbonyl)-cytidine,
5'-désoxy-5-pent-1-ynyl-N⁴-(n-pentyloxycarbonyl)-cytidine,
5'-désoxy-5-hex-1-ynyl-N⁴-n-pentyloxycarbonyl)-cytidine,
5'-désoxy-5-iodo-N⁴-(n-pentyloxycarbonyl)-cytidine,
5-bromo-5'-désoxy-N⁴-(n-pentyloxycarbonyl)-cytidine,
5-(1-chlorovinyl)-5'-désoxy-N⁴-(n-pentyloxycarbonyl)-cytidine,
N⁴-(éthoxycarbonyl)-5'-déscxy-5-vinylcycidine,
5'-désoxy-N⁴-(n-propoxycarbonyl)-5-vinylcytidine,
N⁴-(n-butoxycarbonyl)-5'-déscxy-5-vinylcytidine,
5'-désoxy-N⁴-(n-pentyloxycarbonyl)-5-vinylcytidine,
N⁴-(benzyloxycarbonyl)-5'-désoxy-5-vinylcytidine,
5'-désoxy-N⁴-(n-pentyloxycarbonyl)-5-trifluorométhylcytidine,
5-cyano-5'-désoxy-N⁴-(n-pentyloxycarbonyl)-cytidine,
5'-désoxy-5-éthynyl-N⁴-(méthoxycarbonyl)cytidine,
5'-désoxy-N⁴-(éthoxycarbonyl) -5-éthynylcytidine,
5'-désoxy-5-éthynyl-N⁴-(n-propoxycarbonyl)-cytidine,
5'-désoxy-5-éthynyl-N⁴-(isopropoxycarbonyl)cytidine,
N⁴-(n-butoxycarbonyl)-5'-désoxy-5-éthynylcytidine,
5'-désoxy-5-éthynyl-N⁴-(isobutoxycarbonyl)-cytidine,
5'-désoxy-5-éthynyl-N⁴-(n-pentyloxycarbonyl)-cytidine,
5'-désoxy-5-éthynyl-N⁴-[(2-propylpentyloxy)-carbonyl]-cytidine,
5'-désoxy-5-éthynyl-N⁴-(isopentyloxycarbonyl)-cytidine,
5'-désoxy-5-éthynyl-N⁴-[(2-méthylpentyloxy)-carbonyl]-cytidine,
5'-désoxy-5-éthynyl-N⁴-[(3-méthylpentyloxy)-carbonyl]-cytidine,
5'-désoxy-5-éthynyl-N⁴-(n-hexyloxycarbonyl)-cytidine,
5'-désoxy-N⁴-[(2-éthylbutyl)oxycarbonyl]-5-éthynylcytidine,
5'-désoxy-N⁴-[(2-éthylhexyl)oxycarbonyl]-5-éthynylcytidine,
5'-désoxy-5-éthynyl-N⁴-((2-phényléthoxy)-carbonyl]-cytidine,
N⁴-(cyclohexyloxycarbonyl)-5'-désoxy-5-éthynylcytidine,
N⁴-[(cyclohexylméthoxy)carbonyl]-5'-désoxy-5-éthynyl-cytidine,
5'-désoxy-5-éthynyl-N⁴-(néopentyloxycarbonyl)-cytidine,
5'-désoxy-N⁴-[(3,3-diméthylbutoxy)carbonyl]-5-éthynylcytidine,
2',3'-di-O-acétyl-5'-désoxy-5-éthynyl-N⁴-(n-propoxy-carbonyl)cytidine et
2',3'-di-O-acétyl-5'-désoxy-5-éthynyl-N⁴-(n-pentyloxy-carbonyl)cytidine
2',3'-di-O-acétyl-N⁴-(éthoxycarbonyl)-5'-désoxy-5-vinylcytidine
2',3'-di-O-acétyl-5'-désoxy-N⁴-(n-propoxycarbonyl)-5-vinylcytidine,
2',3'-di-O-acétyl-N⁴-(n-butoxycarbonyl)-5'-désoxy-5-vinylcytidine,
2',3'-di-O-acétyl-5'-désoxy-N⁴-(n-pentyloxycarbonyl)-5-vinylcytidine, et
2',3'-di-O-acétyl-N⁴-(benzyloxycarbonyl)-5'-désoxy-5-vinylcytidine,
5'-désoxy-5-éthynyl-N⁴-(n-decylcxycarboryl)-cytidine,
5'-désoxy-5-éthynyl-N⁴-[(2,6-diméthylcyclchexylcxy)-carbcnyl]cytidine,
5'-désoxy-5-éthynyl-N⁴-(benzyloxycarbonyl)-cytidine,
5'-désoxy-5-éthynyl-N⁴-[(1-isopropyl-2-méthylpropoxy)-carbonyl]cytidine,
5-désoxy-5-éthynyl-N⁴-[(3-méthoxybenzyloxy)-carbonyl]-cytidine.

3. Composé selon la revendication 1 ou 2 à utiliser en thérapie médicale.

4. Composé selon la revendication 1 ou 2 à utiliser dans le traitement des tumeurs.

5. Composition pharmaceutique comprenant un composé selon la revendication 1 ou 2 en tant que principe actif.

6. Composition pharmaceutique pour le traitement des tumeurs comprenant un composé selon la revendication 1 ou 2 et un principe actif.

7. Composition pharmaceutique comprenant un composé selon la revendication 1 ou 2 et du 5-fluorouracil ou l'un de ses dérivés.

8. Composition pharmaceutique comprenant de la 2',3'-di-O-acétyl-5'-désoxy-5-vinylcytidine et du 5-fluoro-uracil ou l'un de ses dérivés.

9. Composition selon la revendication 7 ou 8, dans laquelle le 5-fluorouracil ou son dérivé est choisi dans groupe comprenant les composés suivantes :
5-fluoro-1-(2-tétrahydrofuryl)uracil,
1-(n-hexyloxycarbonyl)-5-fluorouracil,
5'-désoxy-5-fluorouridine,
5'-désoxy-5-fluoro-N⁴-(n-propoxycarbonyl)-cytidine,
N⁴-(n-butoxycarbonyl)-5'-désoxy-5-fluorocytidine,
5'-désoxy-5-fluoro-N⁴-(n-pentyloxycarbonyl)-cytidine,
5'-désoxy-5-fluoro-N⁴-(isopentyloxycarbonyl)-cytidine,
5'-désoxy-5-fluoro-N⁴-(n-hexyloxycarbonyl)-cytidine,
5'--désoxy-N⁴-[(2-éthylbutyl)oxycarbonyl]-5-fluoro-cytidine,
5'-désoxy-5-fluoro-N⁴-[(2-phényléthoxy)carbonyl]-cytidine,
N⁴-[(cyclohexylméthoxy)carbonyl]-5'-désoxy-5-fluoro-cytidine,
5'-désoxy-5-fluoro-N⁴-(néopentyloxycarbonyl)-cytidine,
5'-désoxy-N⁴-[(3,3-diméthylbutoxy)carbonyl]-5-fluoro-cytidine,
5'-désoxy-5-fluoro-N⁴-(3,5-diméthylbenzoyl)-cytidine,
5'-désoxy-5-fluoro-N⁴-(3,5-dichlorobenzoyl)-cytidine, et
2',3'-di-O-acétyl-5'-désoxy-5-fluoro-N⁴-(n-pentyloxy-carbonyl)cytidine.

10. Composition pharmaceutique selon la revendication 7 ou 8 destinée au traitement des tumeurs.

11. Utilisation d'un composé selon la revendication 1 ou 2 pour la fabrication d'un médicament pour le traitement des tumeurs.

12. Trousse comprenant une composition pharmaceutique contenant un composé selon la revendication 1 ou 2 en tant que principe actif et une composition pharmaceutique contenant du 5-fluorouracil ou l'un de ses dérivés en tant que principe actif.

13. Procédé de fabrication d'un composé représenté par la formule générale (I), 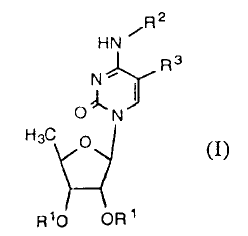
dans laquelle R¹ est un atome d'hydrogène ou un groupe choisi parmi les groupes acétyle, propionyle, benzoyle, toluoyle, glycyle, alanyle, β-alanyle, valyle, lysyle ; R² est un atome d' hydrogène, ou un groupe -CO-OR⁴ [dans lequel R⁴ est un groupe hydrocarboné saturé ou insaturé, à chaîne droite ou ramifiée, comportant de un à quinze atomes de carbone, ou un groupe de formule -(CH₂)ₙ-Y (dans laquelle Y est un groupe cyclohexyle ou phényle ; n est un nombre entier de 0 à 4)] ; R³ est un atome d'hydrogène, de brome, d'iode, ou un groupe trifluorométhyle, éthyle, propyle, cyano, vinyle, 1-chlorovinyle, éthynyle, prop-1-ynyle, but-1-ynyle, pent-1-ynyle, hex-1-ynyle ou bromoéthynyle ; à la condition que R² et R³ ne représentent pas simultanément des atomes d'hydrogène, et que la formule (I) ne représente pas la 2',3'-di-O-acétyl-5'-désoxy-5-vinyl-cytidine, qui consiste :
(A) pour un composé de formule générale (I) dans laquelle R¹, R² et R³ ont les significations données ci-dessus, à faire réagir un composé représenté par la formule (II), 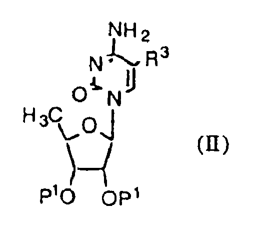 dans laquelle P¹ est un groupe hydroxy-protecteur, et R³ a les significations données ci-dessus,
avec un composé représenté par la formule générale (III),
R⁴OCOX (III)
dans laquelle R⁴ a les significations données ci-dessus ; X est le groupe chloro ou bromo,
en présence d'un accepteur d'acide, puis, si nécessaire, à éliminer le ou les groupes protecteurs,
(B) pour un composé représenté par la formule (I), dans laquelle R¹ et R² ont les significations données ci-dessus et R³ est un groupe vinyle, 1-chlcrovinyle, éthynyle, prop-1-ynyle, but-1-ynyle, pent-1-ynyle, hex-1-ynyle ou bromoéthynyle, à faire réagir un composé représenté par la formule (IV) 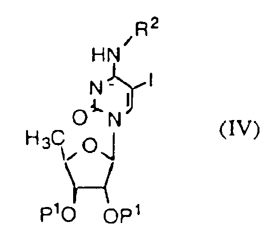 dans laquelle P¹ et R² ont les significations données ci-dessus,
avec un dérivé acétylénique ou vinylique en présence d'un catalyseur au palladium, puis, si nécessaire, à éliminer le ou les groupes protecteurs,
(C) pour un composé représenté par la formule (I) dans laquelle R¹ et R² ont les significations données ci-dessus et R³ est un groupe cyano, à faire réagir un composé représenté par la formule (IV) 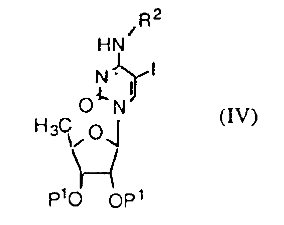 dans laquelle P¹ et R² ont les significations données ci-dessus,
avec un cyanure de métal alcalin, puis, si nécessaire, à éliminer le ou les groupes protecteurs,
(D) pour un composé représenté par la formule (I), dans laquelle R¹ et R³ ont les significations données ci-dessus et R² est un atome d'hydrogène, à faire réagir un composé représenté par la formule (V) 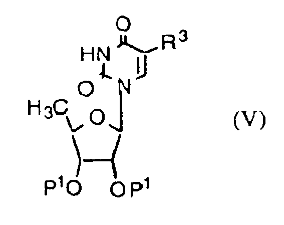 dans laquelle P¹ et R³ ont les significations données ci-dessus,
avec du chlorure de phosphoryle en présence d'un accepteur d'acide, suivi d'un traitement avec de l'ammoniac,
puis, si nécessaire, à éliminer le ou les groupes protecteurs,
(E) pour un composé représenté par la formule (I), dans laquelle R¹, R² et R³ ont les significations données ci-dessus, à coupler un composé représenté par la formule (VI) 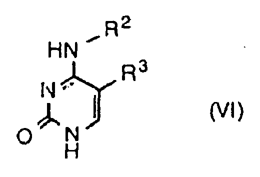 dans laquelle R² et R³ ont les significations données ci-dessus,
avec un composé représenté par la formule (VII) 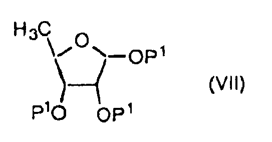 dans laquelle P¹ a les significations données ci-dessus,
en présence d'un catalyseur du type acide Lewis, puis, si nécessaire, à éliminer le ou les groupes protecteurs,
(F) pour un composé représenté par la formule (I) dans laquelle R³ est un groupe vinyle, 1-chlorovinyle, R¹ and R² ont les significations données ci-dessus, à soumettre à une hydrogénation catalytique un composé représenté par la formule (VIII) 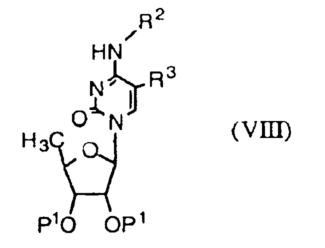 [dans laquelle P¹ est un radical hydroxy-protecteur, R³ est un radical éthynyle ou 1-chloroéthynyle, et R² a les significations données ci-dessus], avec un catalyseur de Lindlar, puis, si nécessaire, à éliminer le ou les radicaux protecteurs.
